# NEUE EUROPÄISCHE PATENTSCHRIFT

(11) **EP 0 765 304 B2**
(45) Veröffentlichungstag und Bekanntmachung des Hinweises auf die Entscheidung über den Einspruch: **22.08.2007**
(45) Hinweis auf die Patenterteilung: 11.08.1999
(21) Anmeldenummer: 95921761.3
(22) Anmeldetag: 26.05.1995
(51) Int. Cl.: C07C 67/22, C07C 249/08, C07C 249/12, C07C 251/48, C07C 69/738, C07C 251/60, C07C 235/78, C07D 249/12

(54) **VERFAHREN UND ZWISCHENPRODUKTE ZUR HERSTELLUNG VON ALPHA-METHOXYIMINOCARBONSÄUREMETHYLAMIDEN**
METHOD OF PREPARING ALPHA-METHOXYIMINOCARBOXYLIC ACID METHYLAMIDES, AND INTERMEDIATES USED IN THE METHOD
PROCEDE DE PREPARATION DE METHYLAMIDES D'ACIDE ALPHA-METHOXYIMINOCARBOXYLIQUE ET INTERMEDIAIRES UTILISES A CET EFFET

(30) Priorität: 10.06.1994 DE 4420416
(43) Veröffentlichungstag der Anmeldung: 02.04.1997
(73) Patentinhaber: Bayer CropScience AG, 40789 Monheim (DE)
(72) Erfinder: BAYER, Herbert, D-68159 Mannheim (DE); ISAK, Heinz, D-67459 Böhl-Iggelheim (DE); WINGERT, Horst, D-68159 Mannheim (DE); SAUTER, Hubert, D-68167 Mannheim (DE); KEIL, Michael, D-67251 Freinsheim (DE); NETT, Markus, D-67105 Schifferstadt (DE); BENOIT, Remy, F-64300 Lanneplaa (FR); MÜLLER, Ruth, D-67159 Friedelsheim (DE)
(86) Internationale Anmeldenummer: PCT/EP1995/002013
(87) Internationale Veröffentlichungsnummer: WO 1995/034526

(56) Entgegenhaltungen:
- EP-A- 0 254 426
- EP-A- 0 299 694
- EP-A- 0 354 571
- EP-A- 0 374 811
- EP-A- 0 460 575
- EP-A- 0 463 488
- EP-A- 0 564 984
- EP-A- 0 585 751
- WO-A-96/37463
- DE-A- 4 042 273
- US-A- 5 157 144
- JOURNAL OF THE AMERICAN CHEMICAL SOCIETY, Bd.113, Nr.4, 1991, WASHINGTON, DC US Seiten 1364 - 73 T. OHWADA ET AL 'Friedel-Crafts-type reactions involving di- and tricationic species. Onium-allyl dications and O,O-diprotonated aci-nitro species bearing a protonated carbonyl group'
- BULLETIN OF THE CHEMICAL SOCIETY OF JAPAN, Bd.58, Nr.9, 1985, TOKYO JP Seiten 2519 - 2522 T. SHIMIZU ET AL. 'A new synthetic method for alkyl carbonocyanidate N-oxides'
- CHEMICAL ABSTRACTS, vol. 100, no. 1, 2. Januar 1984, Columbus, Ohio, US; abstract no. 5665, L. P. SHADRINA ET AL. Seite 485 ; & SINT. METODY OSN. ELEMENTOORG. SOEDIN., 1982 Seiten 41 - 54
- CHEMICAL ABSTRACTS, vol. 96, no. 1, 4. Januar 1982, Columbus, Ohio, US; abstract no. 6320, YU. A. NAUMOV ET AL. Seite 571 ; & KHIM. SREDSTVA ZASHCHITY RAST., M, 1980 Seiten 20 - 26
- CHEMICAL ABSTRACTS, vol. 115, no. 25, 23. Dezember 1991, Columbus, Ohio, US; abstract no. 279536, P. SILVA ET AL. Seite 964 ; & REV. LATINOAM. QUIM., Bd.21, Nr.3-4, 1990 Seiten 108 - 14
- CHEMICAL ABSTRACTS, vol. 67, no. 13, 25. September 1967, Columbus, Ohio, US; abstract no. 64060, N. A. KARANOV ET AL. Seite 6012 ; & SU,A,189 420
- RECUEIL DES TRAVAUX CHIMIQUES DES PAYS-BAS, Bd.56, 1937, DEN HAAG NL Seiten 203 - 207 R. ROGER, F. C. HARPER 'The dehydration of 1-o-tolyl-2,2-diphenylethylene glycol'
- Houben-Weyl, "Methoden der org. Chemie", Alkohole, Bd. 6/1a/1
- Handbook of Chemistry and Physics, 53. Aufl., C-175, C-176, 1972/1973
- J. March, "Advanced Organic Chemistry", McGraw-Hill, S. 813, 1977

## Beschreibung

Die vorliegende Erfindung betrifft ein Verfahren zur Herstellung von α-Methoxyiminocarbonsäuremethylamiden der Formel I, 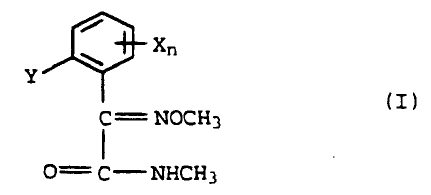 in der
- X: Nitro, Trifluormethyl, Halogen, C₁-C₄-Alkyl oder C₁-C₄-Alkoxy bedeutet,
- n: 0 oder eine ganze Zahl von 1 bis 4 bedeutet, wobei die Reste X verschieden sein können, wenn n > 1 ist, und wobei
- Y: für einen C-organischen Rest steht,
durch Pinner-Umsetzung eines Acylcyanids der Formel II 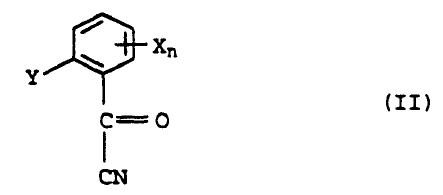
mit einem Alkohol und anschließende Umsetzung des bei der Pinner-Reaktion gebildeten Esters der Formel IV 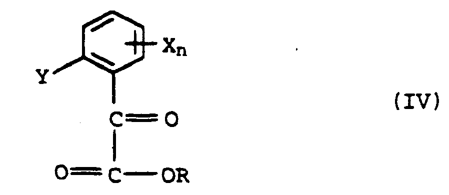
a) mit Hydroxylamin zum Oxim der Formel V, 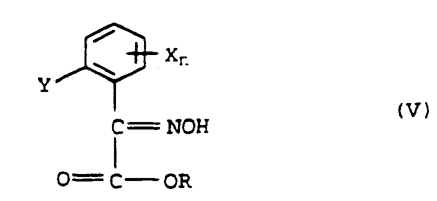 Methylierung von V zum Oximether der Formel VI 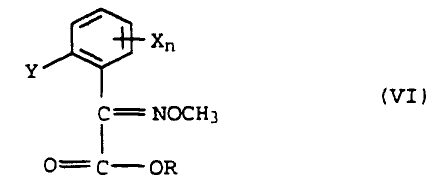 oder
b) mit O-Methylhydroxylamin zum Oximether der Formel VI
   und anschließende Umsetzung von VI mit Methylamin.

Aus der Literatur sind verschiedene Verfahren zur Herstellung von α-Methoxyiminocarbonsäuremethylamidenbekannt. Diese Verfahren sind aber entweder - infolge vieler benötigter Schritte - umständlich und/oder liefern keine befriedigenden Ausbeuten oder sie erfordern den Einsatz teurer oder in großtechnischen Verfahren schwer handhabbare Reagentien (vgl. EP-A 398 692, EP-A 463 488, EP-A 477 631, EP-A 579 124, EP-A 582 925, EP-A 585 751, EP-A 617 011, EP-A 617 014, WO-A 92/13,830, WO-A 93/07,116, WO-A 93/08,180, WO-A 94/08,948, WO-A 94/11,334, WO-A 94/14,322, WO-A 94/14,761, WO-A 94/19,331, WO-A 94/22,812, JP-A 04/182,461, JP-A 05/201,946, JP-A 05/255,012, DE Anm. Nr. 44 10 424.3 und DE Anm. Nr. 44 21 182.1).

Außerdem ist in der Literatur die Pinner-Reaktion von Cyanoketonen der Formel II mit Methanol und die anschließende Umsetzung zu den entsprechenden α-Methoxyiminocarbonsäuremethyletstern l' bekannt (EP-A 493 711). Dieses Verfahren hat jedoch den Nachteil, daß zum einen neben den gewünschten Ketoestern zu einem nicht unerheblichen Teil auch Benzoesäureester, Ketal-Ester und Amide gebildet werden.

Außerdem hat das bekannte Verfahren folgenden Nachteil:

Werden die besonders bevorzugten Verbindungen IIA' (y bedeutet Chlormethyl) 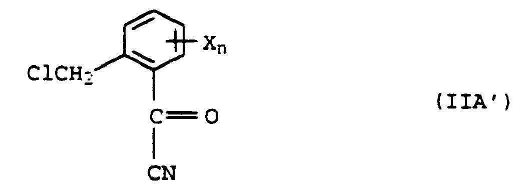 z.B. nach den in DE-A 42 23 382 und DE-A 43 11 722 angegebenen Methoden hergestellt und anschließend mit Methanol zu den Ketoestern X 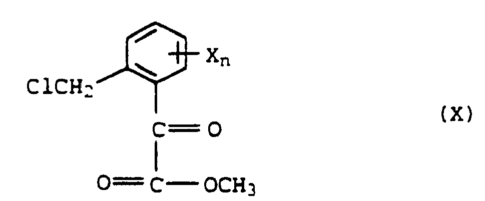 umgesetzt, so bereitet die Reindarstellung von X große Schwierigkeiten. Der Grund hierfür ist, daß die physikalischen Eigenschaften der Ketoester X und der aus den ersten beiden Reaktionen (nach DE-A 42 23 382 und DE-A 43 11 722) mitgeschleppten Nebenprodukte (insbesondere subst. Phthalid und subst. 2-Chlormethylbenzoylchlorid) sehr ähnlich sind, so daß eine Reinigung, z.B. durch Destillation, - wenn überhaupt - nur schwer und mit großem Aufwand möglich ist.

Demgemäß führt die Verwendung der nach dem bekannten Verfahren erhältlichen Ketoestern zu verunreinigten Folgeprodukten, die sich nur schwer aufreinigen lassen.

Der vorliegenden Erfindung lag daher als Aufgabe ein einfaches und großtechnisch anwendbares Verfahren zur Herstellung von α-Methoxyiminocarbonsäureamiden zugrunde, welches insbesondere ohne teure oder bedenkliche Reagentien auskommen sollte und außerdem eine Reindarstellung der gewünschten Zwischen- und Endprodukte erlauben sollte.

Demgemaß wurde ein Verfahren zur Herstellung von α-Methoxyiminocarbonsäuremethylamiden der Formel I, 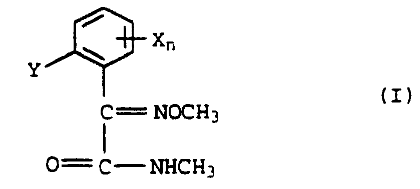 in der
- X: Nitro. Trifluormethyl, Halogen, C₁-C₄-Alkyl oder C₁-C₄-Alkoxy bedeutet,
- n: 0 oder eine ganze Zahl von 1 bis 4 bedeutet, wobei die Reste X verschieden sein können, wenn n > 1 ist, und wobei
- Y: für einen C-organischen Rest steht,
durch Pinner-Umsetzung eines Acylcyanids der Formel II 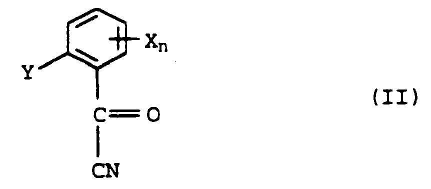
mit einem Alkohol und anschließende Umsetzung des bei der Pinner-Reaktion gebildeten Esters der Formel IV 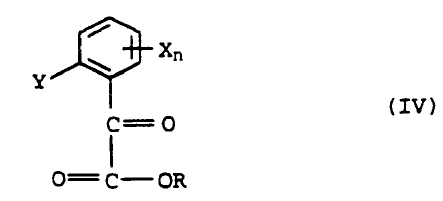
a) mit Hydroxylamin zum Oxim der Formel V, 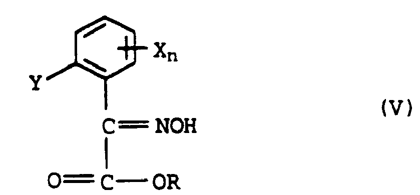 Methylierung von V zum Oximether der Formel VI 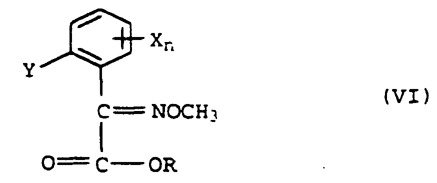 oder
b) mit O-Methylhydroxylamin zum Oximether der Formel VI
   und anschließende Umsetzung von VI mit Methylamin gefunden, welches dadurch gekennzeichnet ist, daß man bei der Pinner-Reaktion einen Alkohol der Formel III

   R-OH (III)

   verwendet, dessen Siedepunkt oberhalb von 75°C liegt.

Das Verfahren beruht im Prinzip darauf, daß durch die Verwendung höhersiedender Alkohole bei der Pinner-Reaktion α-Ketoester gebildet werden, die ebenfalls schwerer flüchtig sind. Dadurch steigt das Siedepunktsintervall zwischen dem gewünschten Produkt und den unerwünschten Nebenprodukten und eine destillative Trennung wird möglich. Bei der Verwendung von höhersiedenden Alkoholen tritt außerdem die Bildung der Nebenprodukte zurück, so daß das gewünschte Produkt selektiver und in besseren Ausbeuten erhalten werden kann.

Bei dem erfindungsgemäßen Verfahren geht man im allgemeinen so vor, daß man eine Mischung aus Alkohol, Säure und ggf. inertem Lösungsmittel bei Temperaturen von -10°C bis 150°C. vorzugsweise 20°C bis 130°C, insbesondere 50°C bis 110°C, mit dem Acylcyanid II versetzt.

Für das erfindungsgemäße Verfahren eignen sich grundsätzlich alle Alkohole, deren Siedepunkt bei Normaldruck oberhalb von 75°C. vorzugsweise oberhalb von 90°C, insbesondere oberhalb von 120°C, liegt. Beispiele für derartige Alkohole sind Ethanol, n-Propanol, iso-Propanol, n-Butanol, sec. -Butanol, iso-Butanol, tert.-Butanol, n-Pentanol und seine Isomere, n-Hexanol und seine Isomere, Heptanol, Oktanol, Nonanol oder Dekanol und die jeweiligen Isomeren Halogenalkohole wie 2-Chlorethanol, 3-Chlorpropanol, 4-Chlorbutanol, 5-Chlorpentanol, 6-Chlorhexanol, 7-Chlorheptanol, 8-Chloroktanol oder 9-Chlornonanol und die jeweiligen Isomere sowie Alkoxyalkanole wie 2-Methoxyethanol, 2-Ethoxyethanol, 3-Methoxypropanol, 3-Ethoxypropanol, 4-Methoxybutano1 4-Ethoxybutanol, 5-Methoxypentanol, 5-Ethoxypentanol, 6-Methoxyhexanol, 6-Ethoxyhexanol, 7-Methoxyheptanol, 7-Ethoxyheptanol, 8-Methoxyoktanol, 8-Ethoxypktanol, 9-Methoxynonanol oder 9-Ethoxynonanol und die jeweiligen Isomeren.

Besonders bevorzugt sind Ethanol, 1-Propanol, 2-Propanol, 1-Butanol, 2-Butanol, 2-Methyl-1-prapanol, 1-Pentanol, 2-Pentanol, 3-Pentanol, 3-Methyl-1-butanol, 2,2-Dimethyl-1-propanol, 1-Methyl-2-butanol, 2-Methyl-1-butanol, 3-Methyl-2-butanol, 1-Hexanol, 2-Methoxyethanol, 2-Ethoxyethanol, 3-Octanol, 1-Heptanol, 1-Octanol und 2-Chlorethanol. Ganz besonders bevorzugt ist n-Pentanol.

Die Menge an eingesetztem Alkohol ist für das erfindungsgemäße Verfahren nicht kritisch. Im allgemeinen verwendet man 1 bis 10 mol III, vorzugsweise 1 bis 5 mol III, insbesondere 1 bis 3 mol III, pro mol eingesetztes Acylcyanid II. Der Alkohol kann auch als Lösungsmittel dienen. In diesem Fall verwendet man mindestens einen Überschuß von 20 mol, vorzugsweise mindestens 10 mol, insbesondere mindestens 5 mol, pro mol Acylcyanid II.

Als Säure können alle gemäß der Literatur für die Pinner Reaktion verwendbaren anorganischen oder organischen Säuren verwendet werden. Bevorzugt finden Mineralsäuren (z.B. Schwefelsäure und Phosphorsäure, insbesondere Halogenwasserstoffsäuren wie Chlorwasserstoff und Bromwasserstoff) Verwendung.

Die Säuren werden im allgemeinen in einem Überschuß von 1 mol bis 5 mol, vorzugsweise 2 mol bis 5 mol, insbesondere 2,5 mol bis 3,5 mol, pro mol Acylcanid II eingesetzt.

Als inerte Lösungsmittel eingnen sich aprotische polare oder unpolare organische Lösungsmittel, beispielsweise Kohlenwasserstoffe (z.B. Pentan, Hexan, Cyclohexan, Petrolether), aromatische Lösungsmittel (z.B. Benzol, Toluol, o-, m- oder p-Xylol, Chlorbenzol, Nitrobenzol und Anisol), halogenierte Kohlenwasserstoffe (z. B. Dichlormethan, Trichlormethan, Tetrachlormethan und 2,2'-Dichlorethan) und Ether (z.B. Diethylether, Diisopropylether, tert.-Butylmethylether, Tetrahydrofuran, Tetrahydropyran, Dioxan oder Anisol), oder Gemische der genannten Lösungsmittel.

Bevorzugt führt man die Pinner-Reaktion in Gegenwart von Wasser durch, wobei üblicherweise Mengen von 0,5 bis 1,5 mol Wasser pro mol Acylcyanid Verwendung finden.

Die Menge an inertem Lösungsmittel ist für das erfindungsgemäße Verfahren nicht kritisch. In der Regel können 2 Gew.-% bis 40 Gew.-% an Lösungsmittel bezogen auf das Acylcyanid II eingesetzt werden.

In der Regel wird die Umsetzung bei Atmosphärendruck oder bei dem Eigendruck des jeweiligen Reaktionsgemisches durchgeführt. Höherer oder niederiger Druck ist auch möglich bietet aber im allgemeinen keinen zusätzlichen Vorteil.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden (ggf. im Wege einer Vordestillation). Sofern die Zwischen- und Endprodukte als Feststoffe erhalten werden, kann die Reinigung auch durch Umkristallisieren oder Digerieren erfolgen.

Die für die Umsetzung benötigten Acylcyanide II sind beispielsweise nach den in DE-A 42 23 382, EP-A 493 711, EP-A 564 984 und in DE-A 43 11 722 beschriebenen Methoden aus den entsprechenden Phathaliden erhältlich. Die Offenbarung dieser Schriften ist hiermit einbezogen.

Bei dem erfindungsgemäßen Verfahren konnte die Bildung von Ketal-Estern der Formel IV" 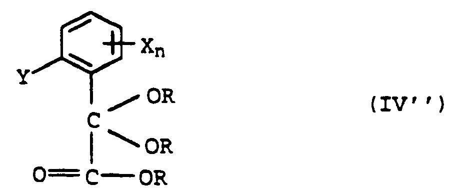 bislang nicht beobachtet werden.

Falls diese Ketale IV" doch einmal als Nebenprodukte auftreten sollten, so wären diese Nebenprodukte jedoch für die weitere Verwendung der Ketoester IV zur Synthese der Verbindungen I nicht störend, da sie unter den Reaktionsbedingungen der anschließenden Reaktion gespalten und mit umgesetzt werden würden. Gewünschtenfalls könnten die Ketocarbonsäureesterdialkylketale IV" aber auch unter sauren Bedingungen, beispielsweise durch Einleiten von Chlorwasserstoff in Gegenwart eines inerten Lösungsmittels, in die Ketoester IV überführt werden.

Desweiteren können bei der Pinnerreaktion die entsprechenden α-Ketocarbonsäureamide IV' gebildet werden. Sind die α-Ketocarbonsäureamide IV' unerwünscht, so unterwirft man zweckmäßigerweise das Rohproduktgemisch erneut der Pinnerreaktion und zwar gegebenenfalls mehrmals, wodurch die α-Ketocarbonsäureamide IV' in die Ketoester IV überführt werden. Die Nebenprodukte der Formel IV' werden nach dem erfindungsgemäßen Verfahren in erheblich geringerem Ausmaß gebildet als dies in bekannten Verfahren der Fall ist.

Die Alkoholyse der α-Ketocarbonsäureamide IV' kann aber auch in einem getrennten Verfahrensschritt erfolgen, beispielsweise durch Behandlung mit Säure und dem Alkohol R-OH, gewünschtenfalls in Gegenwart eines Verdünnungsmittels z. B. eines Kohlenwasserstoffs wie Toluol, eines Halogenkohlenwasserstoffs wie Dichlormethan, Trichlormethan oder Tetrachlorkohlenstoff oder eines Ethers wie Diethylether, Diethylenglycol, Tetrahydrofuran oder Dioxan. Als Säuren kommen beispielsweise Mineralsäuren wie Salzsäure, Schwefelsäure oder Phosphorsäure, Carbonsäuren wie Essigsäure oder Trifluoressigsäure oder Sulfonsäuren wie p-Toluolsuffonsäure in Betracht. Bevorzugte Säuren sind Schwefelsäure, insbesondere als konzentrierte wäßrige Lösung und Salzsäure, die besonders bevorzugt gasförmig eingeleitet wird.

Die Bildung der Oximether VI kann ausgehend von den Ketoestern IV oder den α-Ketocarbonsäureamiden IV' durch Umsetzen mit O-Methylhydroxylamin oder eines seiner Säureadditionsprodukte erfolgen. Darüberhinaus eignen sich auch Gemische dieser Verbindungen als Ausgangsstoffe, wobei auch das durch die Pinnerreaktion erhaltene Rohprodukt-Gemisch ohne weitere Reinigung weiter umgesetzt werden kann.

Das O-Methylhydroxylamin wird entweder in Form eines Säureadditionssalzes oder als freie Base eingesetzt, wobei die unprotonierte Verbindung durch Zugabe einer starken Base aus dem Salz freigesetzt werden kann. Als Salze der O-Methylhydroxylamine kommen die Salze mit ein- bis dreiwertigen Säuren wie insbesondere Salzsäure und Schwefelsäure in Betracht. Die Verwendung von Säureadditionssalzen ist bevorzugt.

Im allgemeinen nimmt man die Reaktion in Gegenwart eines Lösungs- oder Verdünnungsmittels vor. Geeignete Lösungsmittel sind vorzugsweise aromatische Kohlenwasserstoffe wie Benzol, Toluol und o-, m-, p-Xylol, chlorierte Kohlenwasserstoffe wie Methylenchlorid, Alkohole wie Methanol, Ethanol, n-Propanol, n-Pentanol, n-Butanol, 3-Methyl-1-butanol, n-Hexanol und Ether wie Dioxan, Tetrahydrofuran und Diethylether. Besonders bevorzugt sind Methanol, Ethanol oder n-Pentanol.

Die Mengenverhältnisse der Edukte sind nicht kritisch; zweckmäßigerweise setzt man stöchiometrische Mengen an Ausgangsverbindungen ein, sofern sich nicht ein Überschuß der einen oder anderen Komponente, z. B. 10 mol-%, empfiehlt.

Normalerweise liegt die Reaktionstemperatur zwischen 0 und 100°C, vorzugsweise zwischen 20 und 80°C.

Werden u.a. die Amide IV' als Edukte mit eingesetzt, so sollte die Reaktion in Gegenwart des Alkohols R-OH erfolgen.

Eine Verfahrensvariante besteht darin, das aus der Pinnerreaktion erhaltene Rohgemisch ohne Isolierung aus der Reaktionsmischung mit O-Methylhydroxylamin oder einem seiner Säureadditionssalze umzusetzen.

Alternativ dazu ist es auch möglich, die Ketoester IV oder die α-Ketocarbonsäureamide IV' oder ein Gemisch der Verbindungen IV und IV' mit Hydroxylamin oder einem seiner Säureadditionsprodukte zum Oxim V umzusetzen und dieses anschließend ggf. in Gegenwart einer Base und eines geeigneten Lösemittels mit einem Methylierungsmittel zu versetzen.

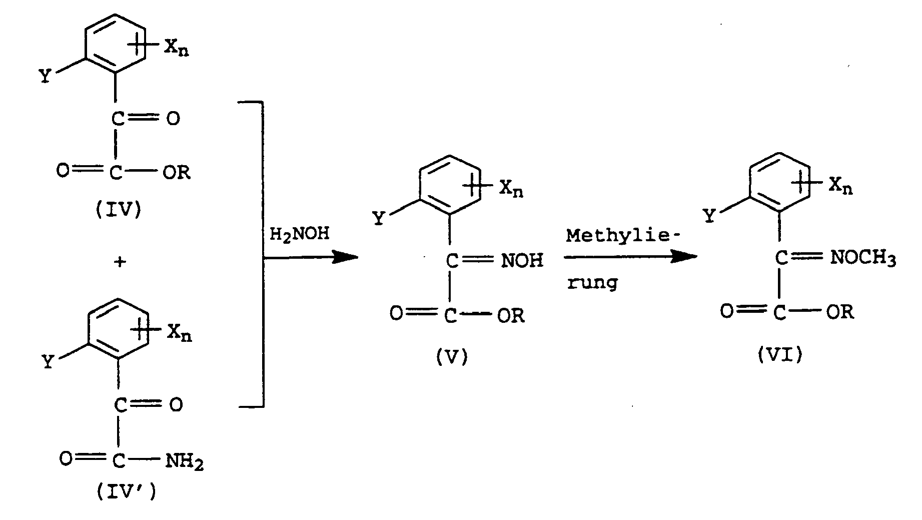

Das Hydroxylamin wird dabei entweder in Form eines Säureadditionssalzes oder als freie Base eingesetzt, wobei die unprotonierte Verbindung durch Zugabe einer starken Base aus dem Salz freigesetzt werden kann. Als Salze des Hydroxylamins kommen die Salze mit ein- bis dreiwertigen Säuren, wie insbesondere Salzsäure und Schwefelsäure, in Betracht. Die Verwendung von Säureadditionssalzen ist bevorzugt.

Beispielsweise nimmt man die Oximbildung in Gegenwart eines Lösungs- oder Verdünnungsmittels vor. Geeignete Lösungsmittel sind vorzugsweise aromatische Kohlenwasserstoffe wie Benzol, Toluol und o-, m-, p-Xylol, chlorierte Kohlenwasserstoffe wie Methylenchlorid, Alkohole wie Methanol, Ethanol, n-Propanol, n-Petnanol, n-Butanol, 3-Methyl-1-butanol und n-Hexanol. Besonders bevorzugt ist Methanol, Ethanol oder n-Pentanol.

Die Mengenverhältnisse der Ausgangsprodukte sind nicht kritisch; zweckmäßigerweise setzt man stöchiometrische Mengen an Ausgangsverbindungen ein, sofern sich nicht ein Überschuß der einen oder anderen Komponente, z. B. 10 mot-% empfiehlt.

Normalerweise liegt die Reaktionstemperatur zwischen 0 und 100°C, vorzugsweise zwischen 20 und 80°C. Werden die Amide IV' als Edukte mit eingesetzt, so muß die Reaktion in Gegenwart des Alkohols R-OH erfolgen. Eine Verfahrensvariante besteht darin, das aus der Pinnerreaktion erhaltene Rohgemisch ohne Isolierung aus der Reaktionsmischung mit Hydroxylamin oder einem seiner Säureadditionsprodukte umzusetzen.

Die Methylierung erfolgt beispielsweise so, daß man die Oxime V in Gegenwart eines Verdünnungsmittels mit einer Base in das entsprechende Salz überführt und dieses mit einem Methylierungsmittel umsetzt. Dabei kann das Oximat vor der Reaktion mit dem Methylierungsmittel isoliert werden oder aber auch direkt weiter umgesetzt werden.

Bevorzugte Basen sind Kaliumhydroxid, Natirumhydroxid, Kaliumcarbonat, Natriumcarbonat, Natriummethylat, Natriumethylat, Natrium-n-pentylat und Kalium-tert.-butylat.

Als Methylierungsmittel eignen sich Methylhalogenide, insbesondere Methylchlorid oder aber Dimethylsulfat.

Als organische Verdünnungsmittel sowohl für die Oximatbildung als auch für die Methylierung sind Lösungsmittel brauchbar, wie zum Beispiel Aceton, Dioxan, Tetrahydrofuran, Alkohole wie Methanol, Ethanol, n-Propanol, n-Pentanol; Sulfoxide wie Dimethylsulfoxid, Diäthylsulfoxid, Dimethylsulfon, Diäthylsulfon, Methyläthylsulfon, Tetramethylensulfon; Nitrile wie Acetonitril, Benzonitril, Butyronitril, lsobutyronitril, m-Chlorbenzonitril; N,N-disubstituierte Carbonamide wie Dimethylformamid, Tetramethylharnstoff, N,N-Dimethylbenzamid, N,N-Dimethylacetamid, N,N-Dimethylphenylacetamid, N,N-Dimethylcyclohexancarbonsäureamid, N,N-Dimethylpropionsäureamid und homologes Carbonsäurepiperidid, Carbonsäuremorpholid, Carbonsäurepyrrolidid; entsprechende N,N-Diäthyl-, N,N-Dipropyl-, N,N-Diisopropyl-, N,N-Diisobutyl-, N,N-Dibenzyl-, N,N-Diphenyl-, N-Methyl-N-phenyl-, N-Cyclohexyl-N-methyl-, N-Äthyl-N-tert.-butyl-verbindungen, N-Methyl-formanilid, N-Äthylpyrrolidon, N-Butylpyrrolidon, N-Äthyl-piperidon-(6), N-Methylpyrrolidon; Hexamethylphosphorsäuretriamid; und entsprechende Gemische. Bevorzugt sind Dimethylacetamid, N-Methylpyrrolidon, Dimethylformamid, Dimethylsulfoxid und Tetramethylensulfon. Besonders bevorzugt N-Methylpyrrolidon und Dimethylformamid.

Die Überführung der Oxime V in ihre Anionen und die anschließende Methylierung wird im allgemeinen bei einer Temperatur von -20 bis 100°C, vorzugsweise von 0 bis 80°C, insbesondere bei 20 bis 80°C durchgeführt.

Das Oxim V, die Base und das Alkylierungsmittel werden in stöchiometrischer Menge eingesetzt oder es wird ein Überschuß der Base und des Alkylierungsmittels, bevorzugt 1,05 bis 1,5 Mol Alkylierungsmittel und 1 bis 1,5 Mol Base je Mol Oxim V verwendet.

Eine Verfahrensvariante besteht darin, daß das Oximsalz ohne Abtrennung des Verdünnungsmittels weiter umgesetzt wird.

Die Oximether VI werden in der Regel als Isomerengemische erhalten, wobei die Oximbindung (C=NOCH₃) teilweise in der E- und teilweise in der Z-Konfiguration vorliegt. Eine Umlagerung der Oximether in die E-Konfiguration ist gewünschtenfalls möglich durch Behandlung des Isomerengemisches von VI in einem organischen Verdünnungsmittel mit einem Katalysator, bevorzugt einer Säure.

Geeignete Lösungsmittel sind vorzugsweise Aceton, aromatische Kohlenwasserstoffe wie Benzol, Toluol und o-, m-, p-Xylol, chlorierte Kohlenwasserstoffe wie Methylenchlorid, Alkohole wie Methanol, Ethanol, n-Propanol, n-Butanol, n-Pentanol, 3-Methyl-1-butanol und n-Hexanol, Ether wie Diethylether, Dioxan, Tetrahydrofuran, tert.-Butylmethylether und Diisopropylether, Sulfoxide wie Dimethylsulfoxid, Diäthylsulfoxid, Dimethylsulfon, Diäthylsulfon, Methyläthylsulfon, Tetramethylensulfon; Nitrile wie Acetonitril, Benzonitril, Butyronitril, Isobutyronitril, m-Chlorbenzonitril; N,N-disübstituierte Carbonamide wie Dimethylformamid, Tetramethylharnstoff, N,N-Dimethylbenzamid, N,N-Dimethylacetamid, N,N-Dimethylphenylacetamid, N,N-Dimethylcyclohexancarbonsäureamid, N,N-Dimethylpropionsäureamid und homologes Carbonsäurepiperidid, Carbonsäuremorpholid, Carbonsäurepyrrolidid; entsprechende N,N-Diathyl-, N,N-Dipropyl-, N,N-Diisopropyl-, N,N-Diisobutyl-, N,N-Dibenzyl-, N,N-Diphenyl-, N-Methyl-N-phenyl-, N-Cyclohexyl-N-methyl-, N-Äthyl-N-tert-butyl-verbindungen, N-Methyl-formanilid, N-Äthylpyrrolidon, N-Butylpyrrolidon, N-Äthyl-piperidon-(6), N-Methylpyrrolidon; Hexamethylphosphorsäuretriamid; und entsprechende Gemische sowie Mischungen mit Wasser.

Besonders bevorzugt sind Methanol, Ethanol, n-Pentanol, Toluol und Diethylether.

Als Säuren eignen sich insbesondere Mineralsäuren, beispielsweise Perchlorsäure, Schwefelsäure, Phosphorsäure und Halogenwasserstoffsäuren wie Chlorwasserstoff, aliphatische Sulfonsäuren wie Trifluormethansulfonsäure, aromatische Sulfonsäuren wie p-Toluolsulfonsäure sowie halogenierte Alkancarbonsäuren wie Trifluoressigsäure. Besonders bevorzugt ist Chlorwasserstoff-Gas.

Üblicherweise verwendet man die 0,01fache bis 10fache, insbesondere die 0,01-bis 5fache, molare Menge an Säure, bezogen auf die Menge des Isomerengemisches VI.

Die Reaktionstemperatur für die Isomerisierung liegt im allgemeinen zwischen (-20) und 100°C, insbesondere zwischen 0 und 80°C.

Die Umlagerung der Oximether benötigt eine gewisse Zeit und zwar abhängig von der Temperatur und insbesondere der Säuremenge etwa 1 bis 90 Stunden, vorzugsweise 2 bis 10 Stunden.

Die Rohlösung nach der Bildung der Oximether VI kann vor dem möglichen Isomerisierungsschritt zunächst eingeengt oder weiterverdünnt werden. Eine bevorzugte Variante besteht darin, daß man die nach der Oximetherbildung erhaltene Rohlösung jedoch ohne weitere Aufkonzentration oder Verdünnung direkt mit der Säure behandelt.

Die so erhaltenen Oximether VI können anschließend mit Methylamin in die entsprechenden α-Methoximinocarbonsäuremethylamide I überführt werden.

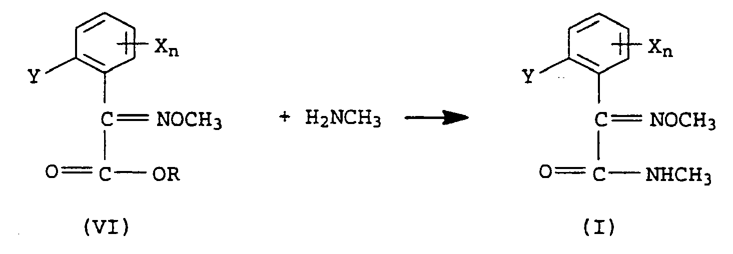

Die Umsetzung erfolgt in an sich bekannter Weise in einem inerten organischen Lösungsmittel bei Temperaturen von 0°C bis 100°C, vorzugsweise 10°C bis 70°C.

Als Lösungsmittel finden insbesondere Acetonitril, Tetrahydrofuran, Dioxan, Methanol, Ethanol, n-Pentanol, N-Methylpyrrolidon, Dimethylformamid, Dimethylacetamid und Dimethylsulfoxid Verwendung.

Methylamin wird üblicherweise in einem Überschuß verwendet, wobei das Methylamin entweder als Gas in die Reaktionsmischung eingeleitet wird oder die Reaktionsmischung mit einer wäßrigen oder alkoholischen Methylaminlösung versetzt wird.

Für den Fall, daß die Amide 1 bei der Herstellung in Form von Isomerengemischen bezüglich der Doppelbindung der Gruppe C=NOCH₃ gebildet werden, können diese gewünschtenfalls gemäß dem in Bezug auf die Oximether VI beschriebenen Verfahren durch Behandlung mit Säuren in die entsprechenden E-Isomere überführt werden.

Das erfindungsgemäße Verfahren eignet sich außerdem zur Herstellung von α-Methoxyiminocarbonsäuremethyletstern der Formel I', 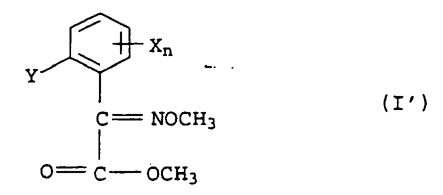 wenn man die Oximether der Formel VI in an sich bekannter Weise umestert (Houben-Weyl, Bd. E5, S. 702-707; Tetrahedron 42, 6719 (1986)).

Die Umesterung wird im allgemeinen wie folgt durchgeführt:

Das Rohprodukt wird in einem Überschuß an Methanol aufgenommen und in bekannter Weise entweder durch Zusatz von Mineralsäuren oder durch Zusatz von Basen (z.B. Natriummethanolat) einer Umesterung unterworfen.

Das erfindungsgemäße Verfahren eignet sich des weiteren insbesondere für die Herstellung von α-Methoxyiminocarbonsäuremethylamiden der Formel IA 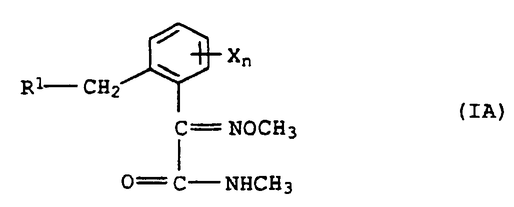 in der die Substituenten und der Index die folgende Bedeutung haben:
- X: Nitro, Trifluormethyl, Halogen, C₁-C₄-Alkyl oder C₁-C₄-Alkoxy,
- n: 0 oder eine ganze Zahl von 1 bis 4, wobei die Reste X verschieden sein können, wenn n > 1 ist,
- R¹: Wasserstoff, Hydroxy, Mercapto, Cyano, Nitro, Halogen, ggf. subst. Alkylsulfonyl, ggf. subst. Alkylsulfonyloxy, ggf. subst. Cycloalkyl, ggf. subst. Aryloxy, ggf. subst. Arylsulfonyl, ggf. subst. Arylsulfonyloxy, ggf. subst. Heterocyclyl oder ggf. subst. Hetaryloxy, 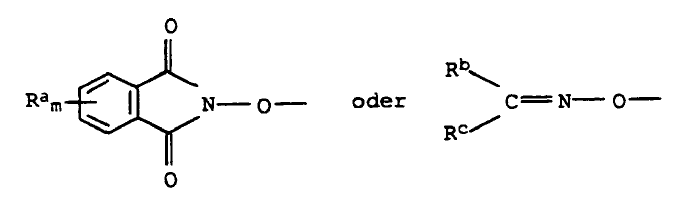
- R^{a}: Cyano, Nitro, Halogen, C₁-C₄-Alkyl, C₁-C₄-Halogenalkyl, C₁-C₄-Alkoxy oder C₁-C₄-Halogenalkoxy,
- m: 0 oder eine ganze Zahl von 1 bis 4, wobei die Reste R^{a} verschieden sein können, wenn m > 1 ist,
- R^{b}: Wasserstoff,
ggf. subst. Alkyl, Cycloalkyl, Alkenyl, Cycloalkenyl, Alkinyl, Heterocyclyl, Alkylcarbonyl, Cycloalkylcarbonyl, Alkenylcarbonyl, Alkinylcarbonyl, Heterocyclylcarbonyl, Alkoxycarbonyl, Aryl, Hetaryl, Arylcarbonyl, Hetarylcarbonyl, Arylsulfonyl, Hetarylsulfonyl oder eine Gruppe C(R')=NOR";
- R': Wasserstoff, Hydroxy, Cyano, Nitro, Amino, Halogen,
ggf. subst. Alkyl, Alkoxy, Alkylthio, Alkylamino, Dialkylamino, Alkenyl, Alkenyloxy, Alkenylthio, Alkenylamino, Alkinyl, Alkinyloxy, Alkinylthio, Alkinylamino, Cycloalkyl, Cycloalkoxy, Cycloalylthio, Cycloalkylamino, Cycloalkenyl, Cycloalkenyloxy, Cycloalkenylthio, Cycloalkenylamino, Heterocyclyl, Heterocyclyloxy, Heterocyclylthio, Heterocyclylamino, Aryl, Aryloxy, Arylthio, Arylamino, Heteroaryl, Heteroaryloxy, Heteroarylthio oder Heteroarylamino;
- R": Wasserstoff,
ggf. sübst. Alkyl, Cycloalkyl, Alkenyl, Alkinyl, Heterocyclyl, Aryl oder Heteroaryl,
- R^{c}: die unter R^{b} genannten Gruppen oder
Hydroxy, Cyano, Nitro, Amino, Halogen,
ggf. subst. Alkoxy, Alkylthio, Alkylamino, Dialkylamino, Aryloxy, Arylthio, Arylamino, Hetaryloxy, Hetarylthio oder Hetarylamino;
oder R^{b} und R^{c} gemeinsam mit dem C-Atom, an das sie gebunden sind, ein carbocyclischer oder heterocyclischer Ring.

Derartige Verbindungen sind aus der eingangs zitierten Literatur als Wirkstoffe zur Bekämpfung von Schadpilzen bekannt.

Das erfindungsgemäße Verfahren eignet sich außerdem zur Herstellung von α-Methoxyminocarbonsäuremethyletsternder Formel l'A 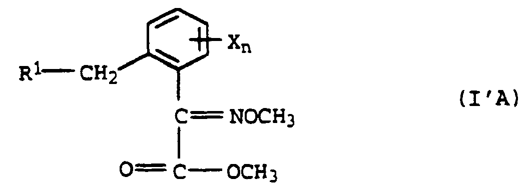 in der die Substituenten und der Index die vorstehend bei den Verbindungen IA gegebene Bedeutung haben. Derartige Verbindungen sind beispielsweise aus EP-A 253 213, EP-A 254 426, EP-A 363 818, EP-A 378 308, EP-A 385 224, EP-A 386 561, EP-A 400 417, EP-A 407 873, EP-A 460, 575, EP-A 463 488, EP-A 472 300, WO-A 94/00,436 und DE Anm. Nr. 44 21 180.5 zur Bekämpfung von Schadpilzen bekannt.

Demgemäß werden besonders Verbindungen der Formel IIA 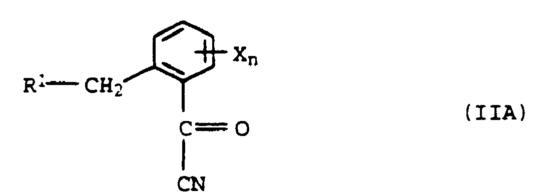 als Ausgangsstoffe bevorzugt.

Für die Herstellung der aus der Literatur bekannten Wirkstoffe ist es unerheblich, ob als Verbindungen IIA diejenigen Substanzen eingesetzt werden, in denen R¹ Wasserstoff, Hydroxy, Mercapto, Cyano, Nitro, ggf. subst. Alkylsulfonyloxy, ggf. subst. Arylsulfonyloxy oder Halogen bedeutet, oder diejenigen, in denen R¹ für ggf. subst. Alkylsulfonyl, ggf. subst. Cycloalkyl, ggf. subst. Aryloxy, ggf. subst. Arylsulfonyl, ggf. subst. Heterocyclyl oder ggf. subst. Hetaryloxy, einen Hydroxy-Phthalimidorest 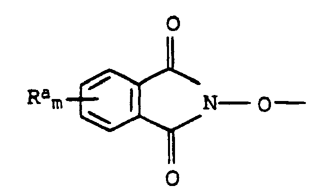 oder einen Oxyiminorest 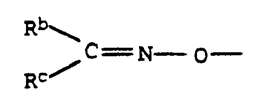 steht. Die in der ersten Gruppe genannten Reste R¹ können bevorzugt auf den Stufen IV und V sowie insbesondere auf den Stufen VI und I gemäß den in der genannten Literatur beschriebenen Verfahren in die Substituenten der zweiten Gruppe überführt werden. Die diesbezüglichen Angaben der zitierten Schriften sind hiermit einbezogen.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl: gesättigte, geradkettige oder verzweigte Kohlenwasserstoffreste mit 1 bis 4, 6 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Alkylcarbonyl: geradkettige oder verzweigte Alkylgruppen mit 1 bis 10 Kohlenstoffatomen (wie vorstehend genannt), welche über eine Carbonylgruppe (-CO-) an das Gerüst gebunden sind;
Alkylsulfonyloxy: geradkettige oder verzweigte Alkylgruppen mit 1 bis 10 Kohlenstoffatomen (wie vorstehend genannt), welche über eine Sulfonyloxygruppe (-SO₂-O-) an das Gerüst gebunden sind;
Halogenalkyl: geradkettige oder verzweigte Alkylgruppen mit 1 bis 4 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkylgruppen mit 1 bis 4 oder 10 Kohlenstoffatomen (wie vorstehend genannt), welche über ein Sauerstoffatom (-O-) an das Gerüst gebunden sind;
Alkoxycarbonyl: geradkettige oder verzweigte Alkoxygruppen mit 1 bis 10 Kohlenstoffatomen (wie vorstehend genannt), welche über eine Carbonylgruppe (-CO-) an das Gerüst gebunden sind;
Halogenalkoxy: geradkettige oder verzweigte Halogenalkylgruppen mit 1 bis 4 Kohlenstoffatomen (wie vorstehend genannt), welche über ein Sauerstoffatom (-O-) an das Gerüst gebunden sind;
Alkylthio: geradkettige oder verzweigte Alkylgruppen mit 1 bis 10 Kohlenstoffatomen (wie vorstehend genannt), welche über ein Schwefelatom (-S-) an das Gerüst gebunden sind;
Alkylamino: eine geradkettige oder verzweigte Alkylgruppe mit 1 bis 4 Kohlenstoffatomen (wie vorstehend genannt), welche über eine Aminogruppe (-NH-) an das Gerüst gebunden ist;
Dialkylamino: zwei voneinander unabhängige geradkettige oder verzweigte Alkylgruppen mit 1 bis 4 Kohlenstoffatomen (wie vorstehend genannt), welche über ein Stickstoffatom (-N:) an das Gerüst gebunden sind;
Alkenyl: ungesättigte, geradkettige oder verzweigte Kohlenwasserstoffreste mit 2 bis 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
Alkenyloxy: eine ungesättigte, geradkettige oder verzweigte Kohlenwasserstoffgruppe mit 2 oder 3 bis 6 oder 10 Kohlenstoffatomen und einer Doppelbindung in einer beliebigen Position (wie vorstehend genannt), welche über ein Sauerstoffatom (-O-) an das Gerüst gebunden ist;
Alkenylthio: eine ungesättigte, geradkettige oder verzweigte Kohlenwasserstoffgruppe mit 2 oder 3 bis 6 oder 10 Kohlenstoffatomen und einer Doppelbindung in einer beliebigen Position (wie vorstehend genannt), welche über ein Schwefelatom (-S-) an das Gerüst gebunden ist;
Alkenylamino: eine ungesättigte, geradkettige oder verzweigte Kohlenwasserstoffgruppe mit 2 oder 3 bis 6 oder 10 Kohlenstoffatomen und einer Doppelbindung in einer beliebigen Position (wie vorstehend genannt), welche über eine Aminogruppe (-NH-) an das Gerüst gebunden ist;
Alkenylcarbonyl: ungesättigte, geradkettige oder verzweigte Kohlenwasserstoffgruppen mit 2 bis 10 Kohlenstoffatomen und einer Doppelbindung in einer beliebigen Position (wie vorstehend genannt), welche über eine Carbonylgruppe (-CO-) an das Gerüst gebunden sind;
Alkinyl: geradkettige oder verzweigte Kohlenwasserstoffgruppen mit 2 bis 10 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl- 1-pentinyl, 3-Methyl-4-pentinyl, 4-Methyl-1-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
Alkinyloxy: eine geradkettige oder verzweigte Kohlenwasserstoffgruppe mit 2 oder 3 bis 6 oder 10 Kohlenstoffatomen und einer Dreifachbindung in einer beliebigen Position (wie vorstehend genannt), welche über ein Sauerstoffatom (-O-) an das Gerüst gebunden ist;
Alkinylthio: eine geradkettige oder verzweigte Kohlenwasserstoffgruppe mit 2 oder 3 bis 6 oder 10 Kohlenstoffatomen und einer Dreifachbindung in einer beliebigen Position (wie vorstehend genannt), welche über ein Schwefelatom (-S-) an das Gerüst gebunden ist;
Alkinylamino: eine geradkettige oder verzweigte Kohlenwasserstoffgruppe mit 2 oder 3 bis 6 oder 10 Kohlenstoffatomen und einer Dreifachbindung in einer beliebigen Position (wie vorstehend genannt), welche über eine Aminogruppe (-NH-) an das Gerüst gebunden ist;
Alkinylcarbonyl: geradkettige oder verzweigte Kohlenwasserstoffgruppen mit 2 bis 10 Kohlenstoffatomen und einer Dreifachbindung in einer beliebigen Position (wie vorstehend genannt), welche über eine Carbonylgruppe (-CO-) an das Gerüst gebunden sind;
Cycloalkyl: monocyclische Alkylgruppen mit 3 bis 12 Kohlenstoffringgliedern, z.B. C₃-C₈-Cycloalkyl wie Cyclopropyl, Cyclobutyl, Cyclopentyl, Cyclohexyl, Cycloheptyl und Cyclooctyl;
Cycloalkoxy: eine monocyclische Alkylgruppe mit 3 bis 6, 8 oder 12 Kohlenstoffringgliedern (wie vorstehend genannt), welche über ein Sauerstoffatom (-O-) an das Gerüst gebunden ist;
Cycloalkylthio: eine monocyclische Alkylgruppe mit 3 bis 6, 8 oder 12 Kohlenstoffringgliedern (wie vorstehend genannt), welche über ein Schwefelatom (-S-) an das Gerüst gebunden ist;
Cycloalkylamino: eine monocyclische Alkylgruppe mit 3 bis 6, 8 oder 12 Kohlenstoffringgliedern (wie vorstehend genannt), welche über eine Aminogruppe (-NH-) an das Gerüst gebunden ist;
Cycloalkylcarbonyl: eine monocyclische Alkylgruppe mit 3 bis 6, 8 oder 12 Kohlenstoffringgliedern (wie vorstehend genannt), welche über eine Carbonylgruppe (-CO-) an das Gerüst gebunden ist;
Cycloalkenyl: monocyclische Kohlenwasserstoffe mit 5 bis 12 Kohlenstoffringgliedern und einer oder zwei Doppelbindungen im Ring, z.B. C₃-C₈-Cycloalkenyl wie Cyclopropenyl, Cyclobutenyl, Cyclopentyl, Cyclohexenyl, Cycloheptenyl, Cyclooctenyl und Cyclohexadienyl;
Cycloalkenyloxy: eine monocyclische Alkenylgruppe mit 5 bis 8 oder 12 Kohlenstoffringgliedern und einer oder zwei Doppelbindungen (wie vorstehend genannt), welche über ein Sauerstoffatom (-O-) an das Gerüst gebunden ist;
Cycloalkenylthio: eine monocyclische Alkenylgruppe mit 5 bis 8 oder 12 Kohlenstoffringgliedern und einer oder zwei Doppelbindungen (wie vorstehend genannt), welche über ein Schwefelatom (-S-) an das Gerüst gebunden ist;
Cycloalkenylamino: eine monocyclische Alkenylgruppe mit 3 bis 8 oder 12 Kohlenstoffringgliedern und einer oder zwei Doppelbindungen (wie vorstehend genannt), welche über eine Aminogruppe (-NH-) an das Gerüst gebunden ist;
Heterocyclyl: ein gesättigter oder partiell ungesättigter cyclischer Rest, welcher neben Kohlenstoffatomen als Ringglieder Heteroatome aus der Gruppe Sauerstoff, Schwefel oder Stickstoff enthält: z.B. 5- oder 6-gliedrige Heterocyclen (Heterocyclyl) enthaltend neben Kohlenstoffringgliedern ein bis drei Stickstoffatome und/oder ein Sauerstoff- oder Schwefelatom oder ein oder zwei Sauerstoff- und/oder Schwefelatome, zwei Sauerstoffund/oder Schwefelatome, z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl,1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 3,4-Isoxazolin-3-yl, 4,5-Isoxazolin-3-yl, 2,3-Isoxazo-lin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isathiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Di-hydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl und 1,2,4-Tetrahydrotriazin-3-yl, besonders bevorzugt 1-Pyrrolidinyl, 1-Pyrazolidinyl, 1-Imidazolidinyl, 2-Isoxazolidinyl, 3-Oxazolidinyl, 2-Isothiazolidinyl, 3-Thiazolidinyl, 2,3-Dihydropyrrol-1-yl, 2,5-Dihydropyrrol-1-yl, 2,3-Dihydropyrazol-1-yl, 4,5-Dihydropyrazol-1-yl, 2,3-Dihydroimidazol-1-yl, 4,5-Dihydroimidazol-1-yl, 2,3-Dihydroisoxazol-2-yl, 2, 3-Dihydrooxazol-3-yl, 2,3-Dihydroisothiazol-2-yl, 2,3-Dihydrothiazol-3-yl, Piperidin-1-yl, Morpholin-1-yl und Pyrazin-1-yl;
Heterocyclyloxy: ein gesättigter oder partiell ungesättigter cyclischer Rest, welcher neben Kohlenstoffatomen als Ringglieder Heteroatome aus der Gruppe Sauerstoff, Schwefel oder Stickstoff enthält (wie vorstehend genannt), welcher über ein Sauerstoffatom (-O-) an das Gerüst gebunden ist;
Heterocyclylthio: ein gesättigter oder partiell ungesättigter cyclischer Rest, welcher neben Kohlenstoffatomen als Ringglieder Heteroatome aus der Gruppe Sauerstoff, Schwefel oder Stickstoff enthält (wie vorstehend genannt), welcher über ein Schwefelatom (-S-) an das Gerüst gebunden ist;
Heterocyclylamino: ein gesättigter oder partiell ungesättigter cyclischer Rest, welcher neben Kohlenstoffatomen als Ringglieder Heteroatome aus der Gruppe Sauerstoff, Schwefel oder Stickstoff enthält (wie vorstehend genannt), welcher über eine Aminogruppe (-NH-) an das Gerüst gebunden ist;
Aryl bzw. Aryloxy, Arylthio, Arylamino, Arylcarbonyl, Arylsulfonyl und Arylsulfonyloxy: aromatische mono- oder polycyclische Kohlenwasserstoffreste welche direkt bzw. (Aryloxy) über ein Sauerstoffatom (-O-) oder (Arylthio) ein Schwefelatom (-S-), (Arylamino) eine Aminogruppe (-NH-), (Arylcarbonyl) über eine Carbonylgruppe (-CO-), (Arylsulfonyl) über eine Sulfonylgruppe (-SO₂-) oder (Arylsulfonyloxy) über eine Sulfonyloxygruppe (-SO₂-O-) an das Gerüst gebunden sind, z. B. Phenyl, Naphthyl und Phenanthrenyl bzw. Phenyloxy, Naphthyloxy und Phenanthrenyloxy und die entsprechenden Thio-, Carbonyl-, Sulfonyl- und Sulfonyloxyreste;
Hetaryl bzw. Hetaryloxy, Hetarylthio, Hetarylamino, Hetarylcarbonyl und Hetarylsulfonyl: aromatische mono- oder polycyclische Reste welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können und welche direkt bzw. (Hetaryloxy) über ein Sauerstoffatom (-O-) oder (Hetarylthio) ein Schwefelatom (-S-), (Hetarylamino) eine Aminogruppe (-NH-), (Hetarylcarbonyl) über eine Carbonylgruppe (-CO-) oder (Hetarylsulfonyl) über eine Sulfonylgruppe (-SO₂-) an das Gerüst gebunden sind, z.B.

- 5-gliedriges Heteroaryl, enthaltend ein bis drei Stickstoffatome: 5-Ring Heteroarylgruppen, welche neben Kohlenstoffatomen ein bis drei Stickstoffatome als Ringglieder enthalten können, z. B. 2-Pyrrolyl, 3-Pyrrolyl, 3-Pyrazolyl, 4-Pyrazolyl, 5-Pyrazalyl, 2-Imidazolyl, 4-Imidazolyl, 1,2,4-Triazol-3-yl und 1,3,4-Triazol-2-yl;
- 5-gliedriges Heteroaryl, enthaltend ein bis vier Stickstoffatome oder ein bis drei Stickstoffatome und ein Schwefel-oder Sauerstoffatom oder ein Sauerstoff oder ein Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder Schwefelatom als Ringglieder enthalten können, z. B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Okadiazol-2-yl, 1,3,4-Thiadiazol-2-yl, 1,3,4-Triazol-2-yl;
- kondensiertes 5-gliedriges Heteroaryl, enthaltend ein- bis vier Stickstoffatome oder ein bis drei Stickstoffatome und/oder ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder ein Schwefelatom als Ringglieder enthalten können, und in welchen zwei benachbarte Kohlenstoffringglieder oder ein Stickstoff- und ein benachbartes Kohlenstoffringglied zu einem aromatischen oder heteroaromatischen Bi- oder Polycyclus verbruckt sein können, z.B. Benzofuranyl, Isobenzofuranyl, Benzothienyl, Isobenzothienyl, Indolyl, Isoindolyl, Benzisoxazolyl, Benzoxazolyl, Benzoisothiazolyl, Benzothiazolyl, Indazolyl, Benzimidazolyl, Pyrrolopyridinyl, Pyrrolopyridazinyl, Pyrrolopyrimidinyl, Pyrrolopyrazinyl, Pyrrolotriazinyl, Furopyridinyl, Furopyridazinyl, Furopyrimidyl, Furopyrazinyl, Furotriazinyl, Thienopyridinyl, Thienopyridazinyl, Thienopyrimidyl, Thienopyrazinyl, Thienotriazinyl, Imidazopyridinyl, Imidazopyridazinyl, Imidazopyrimidyl, Imidazopyrazinyl, Imidazotriazinyl, Pyrazolopyridinyl, Pyrazolopyridazinyl, Pyrazolopyrimidyl, Pyrazolopyrazinyl, Pyrazolotriazinyl, Isoxazolopyridinyl, Isoxazolopyridazinyl, Isoxazolopyrimidyl, Isoxazolopyrazinyl, Isoxazolotriazinyl, Oxazolopyridinyl, Oxazolopyridazinyl, Oxazolopyrimidyl, Oxazolopyrazinyl, Okazolotriazinyl, Isothiazolopyridinyl, Isothiazolopyridazinyl, Isothiazolopyrimidyl, Isothiazolopyrazinyl, Isothiazolotriazinyl, Thiazolopyridinyl, Thiazolopyridazinyl, Thiazolopyrimidyl, Thiazolopyrazinyl, Thiazolotriazinyl, Triazolopyridinyl, Triazolopyridazinyl, Triazolopyrimidyl, Triazolopyrazinyl und Triazolotriazinyl;
- über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, wobei diese Ringe über eines der Stickstoffringglieder an das Gerüst gebunden sind, z.B. 1-Pyrrolyl, 1-imidazolyl, 1-Pyrazolyl und 1,2,4-Triazol-1-yl;
- 6-gliedriges Heteroaryl, enthaltend ein bis drei bzw. ein bis vier Stickstoffatome:
   6-Ring Heteroarylgruppen, welche neben Kohlenstoffatomen ein bis drei bzw. ein bis vier Stickstoffatome als Ringglieder enthalten können, z. B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
- kondensiertes 6-gliedriges Heteroaryl, enthaltend ein_bis vier Stickstoffatome: 6-Ring Heteroarylgruppen in welchen zwei benachbarte Kohlenstoffringglieder zu einem aromatischen oder heteroaromatischen Bi- oder Polycyclus verbrückt sein können, z.B. Chinolin, Isochinolin, Chinazolin und Chinoxalin,
bzw. die entsprechenden Oxy-, Thio-, Amino-, Carbonyl- oder Sulfonylgruppen.

Der Zusatz *"ggf. subst.* "in **Bezug auf** *Alkyl-, Alkylcarbonyl-, Alkylsulfonyl-, Alkoxy-, Alkoxycarbonyl-, Alkylthio-, Alkyl-amino-, Dialkylamino-, Alkenyl-, Alkenyloxy-, Alkenylthio-, Alkenylamino-, Alkenylcarbonyl-, Alkinyl-, Alkinyloxy-, Alkinylthio-, Alkinylamino- und Alkinylcarbonylgruppen* soll zum Ausdruck bringen, daß diese Gruppen partiell oder voll- _ ständig halogeniert sein können und/oder einen bis drei, vorzugsweise einen, der folgenden Reste tragen können: Cyano, Nitro, Hydroxy, Merkapto, Amino, Carboxyl, Aminocarbonyl, Aminothiocarbonyl, Halogen, C₁-C₆-Alkoxy, C₁-C₆-Halogenalkoxy, C₁-C₆-Alkoxycarbonyl, C₃-C₆-Cycloalkyl, C₁-C₆-Alkylamino (eine NH-Gruppe, welche eine Alkylgruppe wie vorstehend genannt trägt), Di-C₁-C₆-alkylamino (eine Aminogruppe, welche zwei voneinander unabhängige Alkylgruppen wie vorstehend genannt trägt), Aryl, Aryloxy, Hetaryl oder Hetaryloxy, Arylthio oder Hetarylthio, wobei die letztgenannten aromatischen bzw. heteroaromatischen Gruppen ihrerseits partiell oder vollständig halogeniert sein können und/oder eine bis drei der folgenden Gruppen tragen können: Cyano. Nitro, Hydroxy, Amino, Carboxyl, Aminocarbonyl, Aminothiocarbonyl, C₁-C₄-Alkyl, C₁-C₄-halogenalkyl, C₁-C₄-Alkoxy, C₁-C₄-Halogenalkoxy, C₁-C₄-Alkylthio, C₁-C₄-Alkylamino, Di-C₁-C₄-alkylamino und C₁-C₄-Alkoxycarbonyl.

Der Zusatz "ggf. subst." zu den genannten cycloalkyl-, Cycloalkenyl-, Heterocyclyl-, Aryl- und Hetarylgruppen (bzw. die entsprechenden Oxy-, Thio-, Carbonyl-, Sulfonyl- und Sulfonyloxygruppen) soll zum Ausdruck bringen, daß diese Gruppen partiell oder vollständig halogeniert sein können und/oder einen bis vier, vorzugsweise einen oder zwei, der folgenden Reste tragen können: Cyano, Nitro, Hydroxy, Merkapto, Amino, Carboxyl, Aminocarbonyl, Aminothiocarbonyl, Halogen, C₁-C₆-Alkyl, C₁-C₆-Halogenalkyl, C₁-C₆-Alkylcarbonyl, C₁-C₆-Alkoxy, C₁-C₆-Halogenalkoxy, C₁-C₆-Alkoxycarbonyl, C₁-C₆-Alkylthio, C₃-C₆-Cycloalkyl, C₁-C₆-Alkylamino (eine NH-Gruppe, welche eine Alkylgruppe wie vorstehend genannt trägt), Di-C₁-C₆-alkylamino (eine Aminogruppe, welche zwei voneinander unabhängige Alkylgruppen wie vorstehend genannt trägt), C₁-C₆-Alkylsulfonyl, C₂-C₆-Alkenyl, C₂-C₆-Alkenyoxy, Aryl, Aryl-C₁-C₄-alkyl, Aryloxycarbonyl, Aryloxy, Hetaryl, Hetaryloxy oder 1-(C₁-C₆-Alkoxyimino)-C₁-C₆-alkyl, wobei die aromatischen bzw. heteroaromatischen Gruppen partiell oder vollständig halogeniert sein können und/oder ein bis drei der folgenden Gruppen t ragen können: Cyano, Nitro, Hydroxy, Amino, Carboxyl, Aminocarbonyl, Aminothiocarbonyl, C₁-C₄-Alkyl, C₁-C₄-Halogenalkyl, C₁-C₄-Alkylcarbonyl, C₁-C₄-Alkoxy, C₁-C₄-Halogenalkoxy, C₁-C₄-Alkoxycarbonyl, C₁-C₄-Alkylthio, C₁-C₄-Alkylamino und Di-C₂-C₄-alkylamino.

Die Angabe "partiell oder vollständig halogeniert" soll zum Ausdruck bringen, daß in den derart charakterisierten Gruppen die an C-Atome gebundenen Wasserstoffatome zum Teil oder vollständig durch gleiche oder verschiedene Halogenatome wie vorstehend genannt, insbesondere Fluor, Chlor und/oder Brom, ersetzt sein können.

Einzelne Zwischenprodukte der Formel IVA, in der R¹ für Wasserstoff steht, sind aus der Literatur bekannt [vgl : Bull. Soc. Chim. Fr. Bd. 17, S. 363 ff (1897); ebd. Bd. 35, S. 202 (1924); Bull. Chem. Soc. Jp. Bd. 57, S. 810 ff. (1984)].

Insbesondere sind Zwischenprodukte der Formeln IVA, IV'A, VA und VIA bevorzugt, in denen R¹ für Chlor steht.

Diese Verbindungen ermöglichen einen leichten Zugang zu den verschiedensten in der Vorliteratur beschriebenen Wirkstoffen.

Stellvertreter der besonders bervorzugten Zwischenprodukte sind in den anschließenden Tabellen zusammengestellt.
Tabelle 1
   Verbindungen der Formel IVA, VA und VIA, in denen Xₙ für Wasserstoff steht. R CH₂CH₂CH₃ bedeutet und R¹ für Chlor steht.
Tabelle 2
   Verbindungen der Formel IVA, VA und VIA. in denen Xₙ für Wasserstoff steht, R (CH₂)₃CH₃ bedeutet und R¹ für Chlor steht.
Tabelle 3
   Verbindungen der Formel IVA, VA und VIA, in denen Xₙ für Wasserstoff steht, R CH₂CH(CH₃)₂ bedeutet und R¹ für Chlor steht.
Tabelle 4
   Verbindungen der Formel IVA, VA und VIA, in denen Xₙ für Wasserstoff steht, R CH(CH₃)CH₂CH₃ bedeutet und R¹ für Chlor Steht.
Tabelle 5
   Verbindungen der Formel IVA, VA und VIA, in denen Xₙ für Wasserstoff steht, R (CH₂)₄CH₃ bedeutet und R¹ für Chlor steht.
Tabelle 6
   Verbindungen der Formel IVA, VA und VIA, in denen Xₙ für Wasserstoff steht, R CH₂C(CH₃)₃ bedeutet und R¹ für Chlor steht.
Tabelle 7
   Verbindungen der Formel IVA, VA und VIA, in denen Xₙ für Wasserstoff steht, R C(CH₃)₂CH₂CH₃ bedeutet und R¹ für Chlor steht.
Tabelle 8
   Verbindungen der Formel IVA, VA und VIA, in denen Xₙ für Wasserstoff steht, R Pent-2-yl bedeutet und R¹ für Chlor steht.
Tabelle 9
   Verbindungen der Formel IVA, VA und VIA, in denen Xₙ für Wasserstoff steht, R Pent-3-yl bedeutet und R¹ für Chlor steht.
Tabelle 10
   Verbindungen der Formel IVA, VA und VIA, in denen Xₙ für Wasserstoff steht, R 2-Methylbut-1-yl bedeutet und R¹ für Chlor steht.
Tabelle 11
   Verbindungen der Formel IVA, VA und VIA, in denen Xₙ für Wasserstoff steht, R 3-Methylbut-2-yl bedeutet und R¹ für Chlor steht.
Tabelle 12
   Verbindungen der Formel IVA, VA und VIA, in denen Xₙ für Wasserstoff steht, R Ethyl bedeutet und R¹ für Chlor steht.
Tabelle 13
   Verbindungen der Formel IVA, VA und VIA. in denen Xₙ für Wasserstoff steht, R 1 -Methylethyl bedeutet und R¹ für Chlor Steht.

### Beispiel 1

### 2-(Chlormethyl)phenylglyoxylsäure-n-pentylester

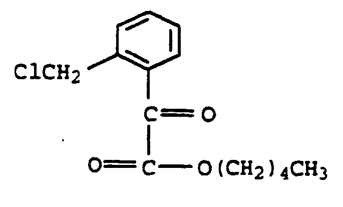

18 g Pentanol-1 (0,2 mol) werden mit 1,8 g Wasser in 80 g Toluol aufgenommen. Man gast nun 14,6 g (0,4 mol) Chlorwasserstoff ein (-0°C). Anschließend tropft man 16,2 g (0,09 mol) 2-(Chlormethyl)benzoylcyanid zu. Man läßt bei Raumtemperatur 2 h nachrühren und erwärmt 8 h bei 60°C.

Das Reaktionsgemisch läßt man abkühlen, extrahiert einmal mit 50 ml 15 %iger Salzsäure und 3mal mit 50 ml Wasser und engt zur Trockene ein.
Ausbeute: 23 g (95 % enthält 4,9 % 2-(Chlormethyl)benzoesäurepentylester)

Kleinere Mengen werden chromatographisch gereinigt, z. B. Cyclohexan : Toluol = 2 : 1 über Kieselgel 60 (Flash).

Größere Mengen werden destillativ gereinigt.
¹H-NMR (CDCl₃): δ = 0,92 (t, 3H); 1,28-1,48 (m, 4H); 1,67-1,84 (m, 2H); 4,39 (t, 2H); 5,03 (s, 2H); 7,45-7,78 (m, 4H) ppm.

### Beispiel 2

### 2-(Chlormethyl)phenylglyoxylsäure- (2-ethyl)-hexylester

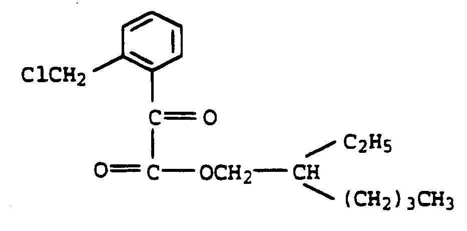

30 g 2-Ethyl-hexanol (0,23 mol) werden mit 2,0 g Wasser in 80 g Toluol gelöst. Man gast bei 0-5°C 14,6 g (0,4 mol) Chlorwasserstoff ein und tropft anschließend 18 g 2-(Chlormethyl)benzoylcyanid gelöst in 25 g Toluol bei 0°C hinzu.

Das Reaktionsgemisch wird innerhalb von 2 h unter Rühren auf 60°C erwärmt. Nach 8 h bei dieser Temperatur läßt man auf Raumtemperatur abkühlen und wäscht 1x mit 50 ml 15 %iger Salzsäure und 3x mit 50 ml Wasser.

### Rohprodukt: 33 g (ca. 88 %ig)

Flash-Chromatographie mit Cyclohexan (3) : Toluol (1) auf Kieselgel 60.
Ausbeute: 23 g (75 %, Reinheit > 99 %)

### Beispiel 3

### 2-Methoxyimino-2-[(2'-chlormethyl)phenyl]essigsäure-n-pentylester

Zur Lösung von 27 g (0,1 mol) 2-(Chlormethyl)phenylglyoxylsäure-n-pentylester in 50 ml Methanol gab man 33 g (0,4 mol) O-Methylhydroxylamin Hydrochlorid sowie 10g trockene Molekularsiebperlen (3 Ä) und ließ 16 Stunden bei Raumtemperatur stehen. Nach Abfiltrieren des Molekularsiebes wurde die Lösung eingeengt, der Rückstand zwischen Methyl-tert. -butylether und Wasser verteilt, die organische Phase mit Wasser gewaschen, über Natriumsulfat getrocknet und eingeengt. Man erhielt 30 g (100 %) der Titelverbindung als hellgelbes Öl, das als Isomerengemisch E:Z = 1:1 vorliegt. Die Trennung der Isomeren ist durch Säulenchromatographie an Kieselgel (Methyl-tert.-butylether/n-Hexan) möglich.
E-Isomer: (farbloses Öl)
   ¹H-NMR (CDCl₃) : δ = 0,87 (t, 3H); 1,20-1,37 (m, 4H); 1,62-1,74 (m, 2H); 4,05 (s, 3H); 4,27 (t, 2H); 4,44 (s, 2H); 7,16 (dd, 1 H); 7,32-7,51 (m, 3H) ppm.
Z-Isomer: (farbloses Öl)
   ¹H-NMR (CDCl₃) : δ = 0,89 (t, 3H); 1,24-1,41 (m, 4H); 1,66-1,77 (m, 2H); 4,04 (s, 3H); 4,30 (t, 2H); 4,88 (s, 2H); 7,32-7,47 (m, 3H); 7,58 (d, 1 H) ppm.

### Beispiel 4

### (E)-2-Methoxyimino-2-[(2'-chlormethyl)-phenyl]essigsäure-n-pentylester

Eine Lösung von 30 g (0,1 mol) 2-Methoxyimino-2-[(2'-chlor-methyl)phenyl]essigsäure-n-pentylester (E:Z = 1:1) in 500 ml Diethylether wurde unter Eiskühlung mit Chlorwasserstoffgas gesättigt. Man ließ den Ansatz auf Raumtemperatur kommen und 16 Stunden bei Raumtemperatur rühren. Nach Einengen und säulenchromatographischer Reinigung an Kieselgel (Methyl-tert.-butyl-ether/n-Hexan) erhielt man 24,3 g (81 % Ausbeute) der gewünschten Titelverbindung als farbloses Öl.
¹H-NMR: siehe Beispiel 1 (E-Isomer).

### Beispiel 5

### (E,E)-2-Methoxyimino-2-{[2'-(1 "-(4"'-chlorphenyl)-1 "-methyl)-iminooxyethyl ]phenyl}essigsäure-n-pentylester

0,27 g (11 mmol) Natriumhydrid wurden in 50 ml Dimethylformamid vorgelegt. Man gab 1,7 g 4-Chloracetophenonoxim portionsweise zu und rührte 30 Minuten bei Raumtemperatur. Anschließend tropfte man 3,0 g (10 mmol) (E)-2-Methoxyimino-2-[(2'-chlormethyl)-phenyl]essigsäure-n-pentylester in 10 ml Dimethylformamid zu und ließ 2 Stunden bei Raumtemperatur rühren. Man goß auf kalte 2M Salzsäure und extrahierte mit Methyl-tert.-butyl-ether. Die vereinigten organischen Phasen wurden mit Wasser gewaschen, über Na₂SO₄ getrocknet und eingeengt. Nach säulenchromatographischer Reinigung an Kieselgel (Methyl-tert.-butylether/n-Hexan) erhielt man 3,5 g (80%) der Titelverbindung als farbloses Öl.
¹H-NMR (CDCl₃) : δ = 0,84 (t, 3H); 1,16-1,36 (m, 4H); 1,53-1,72 (m, 2H); 2,18 (s, 3H) ; 4,02 (s, 3H) ; 4,19 (t, 2H); 5,12 (s, 2H); 7,17-7,59 (m, 8H) ppm.

### Beispiel 6

### (E,E)-2-Methoxyimino-2-{[2'-(1"-(4"'-chlorphenyl)-1"-methyl) - iminooxymethyl]phenyl}essigsäuremonomethylamid

2,0 g (4,6 mmol) (E,E)-2-Methoxyimino-2-{[2'-(1"-(4"'-chlorphenyl)-1"-methyl)iminooxyethyl]pheny})essigsäure-n-pentylester wurden in 50 ml Tetrahydrofuran gelöst, mit 20 ml 40 %iger wäßriger Monomethylaminlösung versetzt und 3 Stunden bei Raumtemperatur gerührt. Anschließend wurde mit Wasser versetzt und mit Methyl-tert.-butylether extrahiert. Die vereinigten organischen Phasen wurden mit Wasser gewaschen, über Natriumsulfat getrocknet und eingeengt. Man erhielt so 1,6 g (92 %) der Titelverbindung als weißes Pulver vom Schmelzpunkt 117-119°C.
¹H-NMR (CDCl₃) : δ = 2,17 (s, 3H); 2,86 (d, 3H); 3,94 (s, 3H); 5,11 (s, 2H); 6,72 (s, br, 1 H); 7,19-7,55 (m, 8H) ppm.

### Beispiel 7

### (Z)-2-Methoxyimino-2-{[2'-(E)-(1 "-(4"'-chlorphenyl)-1 "-methyl)-iminooxymethyl]phenyl}essigsäuremonomethylamid

0,09 g (3,7 mmol) Natriumhydrid wurden in 10 ml Dimethylformamid vorgelegt. Man gab 0,58 g 4-Chloracetophenonoxim protionsweise zu und rührte 30 Minuten bei Raumtemperatur. Anschließend tropfte man 1,0 g (3,4 mmol) (Z)-2-Methoxyimino-2-[(2'-chlormethyl)-phenyl]essigsäure-n-pentylester in 10 ml Dimethylformamid zu, ließ 30 Minuten bei Raumtemperatur rühren, versetzte mit 10 ml Tetrahydrofuran und 10 ml 40 %iger waßriger Monomethylaminlösung und ließ 16 Stunden bei Raumtemperatur rühren. Nach Versetzen mit Wasser wurde mit Methyl-tert.-butylether extrahiert. Die vereinigten organischen Phasen wurden mit Wasser gewaschen, über Natriumsulfat getrocknet und eingeengt. Nach säulenchromatographischer Reinigung an Kieselgel (Methyl-tert.-butyl-ether/n-Hexan) erhielt man 1,0 g (79% Ausbeute) der Titelverbindung als beigefarbenes Pulver vom Schmelzpunkt 111-113°C.
¹H-NMR (CDCl₃) : δ = 2,23 (s, 3H); 2,80 (d, 3H); 4,04 (s, 3H); 5,39 (s, 2H); 6,68 (s, br, 1 H); 7,30-7,55 (m, 8H) ppm.

### Beispiel 8

### (E,E)-2-Methoxyimino-2-[2'-(1"-(4"'-chlorphenyl)-1"-methyl)-iminooxymethyl]phenyl}essigsäuremonomethylamid

Zu einer Lösung von 8,4 g (0,022 mol) (Z)-2-Methoxyimino-2-{[2'-(E)-(1"-(4"'-chlorphenyl)-1"-methyl)iminooxymethyl]phenyl}essigsäuremonomethylamid in 300 ml Toluol gab man 50 ml einer gesättigten etherischen Chlorwasserstofflösung und ließ bei Raumtemperatur 4 Stunden stehen. Nach Zusatz von Methyltert.-butylether wurde mit gesättigter NaHCO₃-Lösung und anschließend mit Wasser neutralgewaschen, die organische Phase abgetrennt, über Na₂SO₄ getrocknet und eingeengt. Nach säulenchromatographischer Reinigung an Kieselgel (Methyl-tert.-butylether/n-Hexan) erhielt man 5,4 g (65 % Ausbeute) der Titelverbindung als farblose Kristalle vom Schmelzpunkt 117 bis 119°C.
¹H-NMR (CDCl₃) : δ = 2,17 (s, 3H); 2,86 (d, 3H); 3,94 (s, 3H); 5,11 (s, 2H); 6,71 (sbr, 1H): 7,19-7,55 (m, 8H) ppm.

### Beispiel 9

### 2-(Chlormethyl)phenylglyoxylsäureamid

16,5 g (92 mmol) 2-(Chlormethyl)benzoylcyanid, 150 ml konzentrierte Salzsäure und 150 ml gesättigte etherische Chlorwasserstofflösung wurden zusammengegeben und 5 Stunden bei Raumtemperatur gerührt. Anschließend wurde auf Wasser gegossen, die organische Phase abgetrennt und die wäßrige Phase mit Methyl-tert.-butylether extrahiert. Die vereinigten organischen Phasen wurden mit Wasser gewaschen, über Natriumsulfat getrocknet und eingeengt. Nach säulenchromatographischer Reinigung an Kieselgel (Methyl-tert.-butylether/n-Hexan) erhielt man 13,4 g (74% Ausbeute) der Titelverbindung als beigefarbenes Pulver vom Schmelzpunkt 105-107°C.
¹ H-NMR (CDCl₃) : δ = 4,90 (s, 2H); 5,79 (s, br, 1H); 7,03 (s, br, 1 H); 7,46-7,69 (m, 3H); 8,02 (d, 1 H) ppm.

### Beispiel 10

### 2-(Chlormethyl)phenylglyoxysäure-n-pentylester

1,5g (7,6 mmol) 2-(Chlormethyl)-phenylglyoxylsäureamid wurde in 200 ml n-Pentanol vorgelegt. Anschließend wurde Chlorwasserstoffgas bis zur Sättigung eingegast, wobei sich die Temperatur bis auf 80°C erwärmte. Anschließend wurde noch 3 Stunden nachgerührt. Man engte den Ansatz ein, versetzte den Rückstand mit Wasser und extrahierte mit Methyl-tert.-butylether. Die vereinigten organischen Phasen wurden mit Wasser gewaschen, über Natriumsulfat getrocknet und eingeengt. Nach säulenchromatographischer Reinigung an Kieselgel (Methyl-tert.-butylether/n-Hexan) erhielt man 1,1 g (54 % Ausbeute) der Titelverbindung als farbloses Öl.
¹H-NMR (CDCl₃) : δ = 0,92 (t, 3H); 1,28-1,48 (m, 4H); 1,67-1,84 (m, 2H); 4,39 (t, 2H); 5,03 (s, 2H); 7,45-7,78 (m, 4H) ppm.

### Beispiel 11

### (E,E,E)-2-[[[[2-(Methoxyimino)-1,2-(dimethyl)ethyliden]amino]-oxy]methyl]-α-methoxyimino-phenylessigsäuremonomethylamid

Insgesamt 1,4 g (10 mmol) Kaliumcarbonat und 0,7 g (5,4 mmol) (E,E)-2-Hydraxyimino-3-methoxyimino-butan wurden in 15 ml Dimethylformamid vorgelegt und 1 h bei 50°C gerührt. Anschließend wurden 1,5 g (5,0 mmol) (E)-2-Methoxyimino-2-[2-chlormethyl)-phenyl]essigsäure-n-pentylester, gelöst in 5 ml Dimethylformamid zugegeben und insgesamt 48 h bei Raumtemperatur gerührt. Man gab nun 20 ml 40 %ige wäßrige Monomethylaminlosung zu und ließ 1 h bei Raumtemperatur nachrühren. Nach Versetzen mit Wasser wurde mit Methyl-tert.-butylether extrahiert. Die vereinigten organischen Phasen wurden mit Wasser gewaschen, über Natriumsulfat getrocknet und eingeengt. Nach säulenchromatographischer Reinigung an Kieselgel (Methyl-tert.-butyl-ether/n-Hexan) erhielt man 1,5 g (91 % Ausbeute) der Titelverbindung als weißes Pulver vom Schmelzpunkt 67 bis 69°C.
¹H-NMR (CDCl₃) : δ = 1,95 (s, 3H); 1,98 (s, 3H); 2,90 (d, 3H); 3,92 (s, 3H); 3,94 (s, 3H); 5,05 (s, 2H); 6,70 (s, br, 1 H); 7,13-7,45 (m, 4H) ppm.

### Beispiel 12

### (E,E,E)-2-[[[[2-(Methoxyimino)-1-(methyl)-2-(phenyl)ethyliden]-amino]oxy]methyl]-α-methoxyimino-phenylessigsäuremonomethylamid

Insgesamt 2,2 g (16 mmol) Kaliumcarbonat und 0,65 g (3,4 mmol) (E,E)-1-Phenyl-1-methoxyimino-propan-2-on-2-oxim wurden in 30 ml Dimethylformamid vorgelegt und 1 Stunde bei 60°C gerührt Anschließend wurden 1,0 g (3,4 mmol) (E)-2-Methoxyimino-2-[(2-chlormethyl)phenyl]essigsäure-n-pentylester, gelöst in 20 ml Dimethylformamid, zugegeben und 28 Stunden bei Raumtemperatur sowie 17 Stunden bei 60°C gerührt. Nach dem Abkühlen gab man nun 50 ml Tetrahydrofuran sowie 15 ml 40 %ige wäßrige Monomethylaminlösung zu und ließ 24 Stunden bei Raumtemperatur nachrühren. Nach Versetzen mit Wasser wurde mit Methyl-tert.-butylether extrahiert. Die vereinigten organischen Phasen wurden mit Wasser gewaschen, über Natriumsulfat getrocknet und eingeengt. Nach säulenchromatographischer Reinigung an Kieselgel (Methyl-tert. -butylether/n-Hexan) erhielt man 1,0 g (75 % Ausbeute) der Titelverbindung als weißes Pulver vom Schmelzpunkt 127 bis 130°C.
¹H-NMR (CDCl₃) : δ = 2,10 (s, 3H); 2,84 (d, 3H); 3,87 (s, 3H); 3,89 (s, 3H) ; 4,91 (s, 2H) ; 6,62 (s, br, 1 H) ; 7,12-7,33 (m, 9H) ppm.

### Beispiel 13

### (E)-2-[[[1-Phenyl-1,2,4-triazol-3-yl]oxy]methyl]-α-methoxyiminophenylessigsäure-n-pentylester

0,80 g (5,0 mmol) 3-Hydroxy-1-Phenyl-1,2,4-triazol und 3,5 g (25 mmol) Kaliumcarbonat wurden in 40 ml Dimethylformamid vorgelegt und 10 Minuten bei Raumtemperatur gerührt. Anschließend gab man 1,5 g (5,0 mmol) (E)-2-Methoxyimino-2-[(2-chlormethyl)-phenyl]essigsäure-n-pentylester, gelöst in 10 ml Dimethylformamid, sowie eine Spatelspitze Kaliumiodid zu und erwärmte 6 Stunden auf 100°C. Nach Versetzen mit Wasser wurde mit Methyl-tert.butylether extrahiert. Die vereinigten organischen Phasen wurden mit Wasser gewaschen, über Natriumsulfat getrocknet und eingeengt. Nach säulenchromatographischer Reinigung an Kieselgel (Methyl-tert. -butyl-ether/n-Hexan) erhielt man 1,7 g (80 % Ausbeute) der Titelverbindung als gelbes Öl.
¹H-NMR (CDCl₃): δ = 0,83 (t, 3H); 1,21-1,32 (m, 4H); 1,60-1,71 (m, 2H); 4,04 (s, 3H); 4,23 (t, 2H); 5,26 (s, 2H); 7,17-7,70 (m, 9H); 8,25 (s, 1 H) ppm.

### Beispiel 14

### (E)-2-[[[1-Phenyl-1,2,4-triazol-3-yl]oxy]methyl)α-methoxyiminophenylessigsäuremonomethylamid

1,5 g (3,6 mmol) des Pentylesters aus Beispiel 13 wurden in 50 ml Tetrahydrofuran gelöst, mit 10 ml 40 %iger wäßriger Monomethylaminlösung versetzt und 16 Stunden bei Raumtemperatur gerührt. Anschließend wurde mit Wasser versetzt, mit Methyl-tert. -butylether extrahiert, die organische Phase mit Wasser gewaschen, über Natriumsulfat getrocknet und einrotiert. Als Rückstand verblieben 1,1 g (86 % Ausbeute) der Titelverbindung als gelbes Öl.
¹H-NMR (CDCl₃): δ = 2,90 (d, 3H); 3,96 (s, 3H); 5,30 (s, 2H); 6,87 (s, br, 1 H); 7,25-7,68 (m, 9H); 8,21 (s, 1 H) ppm.

## Patentansprüche

1. Verfahren zur Herstellung von α-Methoxyiminocarbonsäuremethylamiden der Formel I, 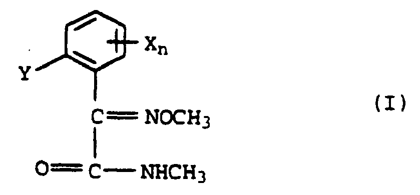 in der
X Nitro, Trifluormethyl, Halogen, C₁-C₄-Alkyl oder C₁-C₄-Alkoxy bedeutet,
n 0 oder eine ganze Zahl von 1 bis 4 bedeutet, wobei die Reste X verschieden sein können, wenn n > 1 ist, und wobei
Y für einen C-organischen Rest steht,
durch Pinner-Umsetzung eines Acylcyanids der Formel II 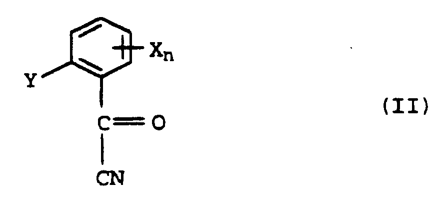
mit einem Alkohol und anschließende Umsetzung des bei der Pinner-Reaktion gebildeten Esters der Formel IV 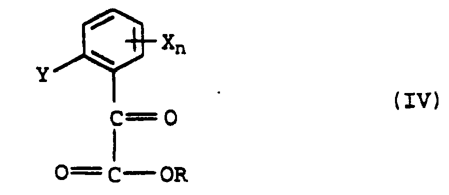
a) mit Hydroxylamin zum Oxim der Formel V, 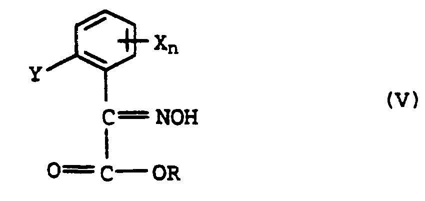 Methylierung von V zum Oximether der Formel VI 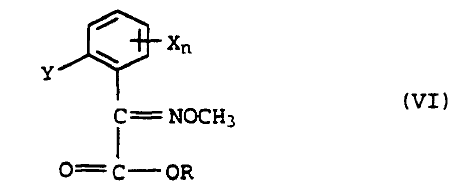 oder
b) mit O-Methylhydroxylamin zum Oximether der Formel VI und anschließende Umsetzung von VI mit Methylamin, **dadurch gekennzeichnet, daß** man bei der Pinner-Reaktion einen Alkohol der Formel III
R-OH (III)
verwendet, dessen Siedepunkt oberhalb von 75°C liegt.

2. Verfahren zur Herstellung von α-Methoxyiminocarbonsäuremethylamiden der Formel I gemäß Anspruch 1, durch Pinner-Umsetzung eines Acylcyanids der Formel II 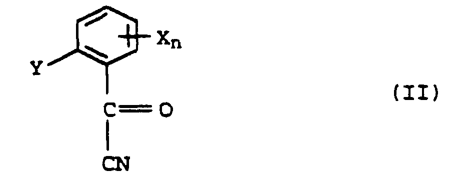 mit einem Alkohol und anschließende Umsetzung des bei der Pinner-Reaktion gebildeten Gemisches aus dem Ester der Formel IV 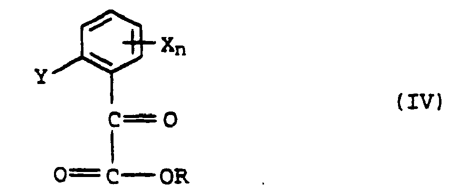 und dem Amid der Formel IV' 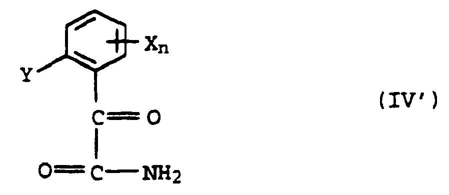
a) mit Hydroxylamin zum Oxim der Formel V, 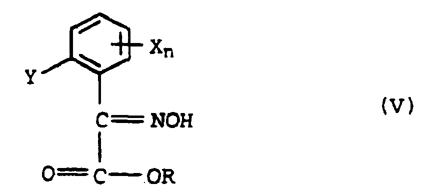 Methylierung von V zum Oximether der Formel VI 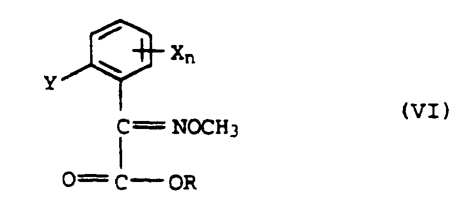 oder
b) mit O-Methylhydroxylamin zum Oximether der Formel VI und anschließende Umsetzung von VI mit Methylamin, **dadurch gekennzeichnet, daß** der man bei der Pinner-Reaktion einen Alkohol der Formel III
R-OH (III)
verwendet, dessen Siedepunkt oberhalb von 75°C liegt.

3. Verfahren zur Herstellung von α-Methoxyiminocarbonsäuremethylamiden der Formel I nach Anspruch 1 oder 2, **dadurch gekennzeichnet, daß** man die Umsetzung zum Oxim der Formel V in Gegenwart desjenigen Alkohols III durchfuhrt, der bei der Pinner-Reaktion verwendet wurde.

4. Verfahren zur Herstellung von α-Methoxyiminocarbonsäuremethylamiden der Formel I nach Anspruch 1 oder 2, **dadurch gekennzeichnet, daß** man die Umsetzung zum Oximether der Formel VI in Gegenwart desjenigen Alkohols III durchfuhrt, der bei der Pinner-Reaktion verwendet wurde.

5. Verfahren nach den Ansprüchen 1 bis 4, **dadurch gekennzeichnet, daß** man einen Alkohol III verwendet, dessen Siedepunkt oberhalb von 90°C liegt.

6. Verfahren zur Herstellung von α-Methoxyiminocarbonsäuremethylamiden der Formel IA 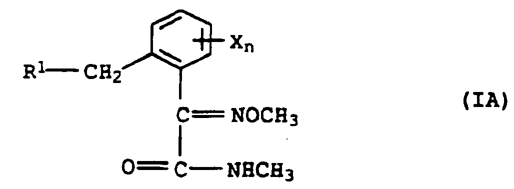 in der die Substituenten und der Index die folgende Bedeutung haben:
X Nitro, Trifluormethyl, Halogen, C₁-C₄-Alkyl oder C₁-C₄-Alkoxy,
n 0 oder eine ganze Zahl von 1 bis 4, wobei die Reste X verschieden sein können, wenn n > 1 ist,
R¹ Wasserstoff, Hydroxy, Mercapto, Cyano, Nitro, Halogen, ggf. subst. Alkylsulfonyl, ggf. subst. Alkylsulfonyloxy, ggf. subst. Cycloalkyl, ggf. subst. Aryloxy, ggf. subst. Arylsulfonyl, ggf. subst. Arylsulfonyloxy, ggf. subst. Heterocyclyl oder ggf. subst. Hetaryloxy, 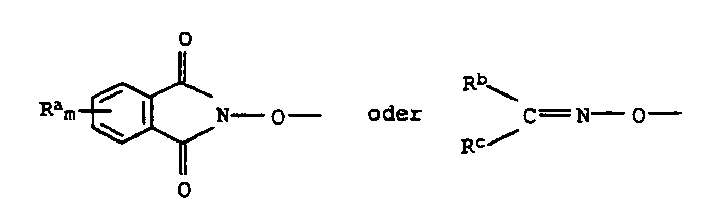
R^{a} Cyano, Nitro, Halogen, C₁-C₄-Alkyl, C₁-C₄-Halogenalkyl, C₁-C₄-Alkoxy oder C₁-C₄-Halogenalkoxy,
m 0 oder eine ganze Zahl von 1 bis 4, wobei die Reste R^{a} verschieden sein können, wenn m > 1 ist,
R^{b} Wasserstoff,
ggf. subst. Alkyl, Cycloalkyl, Alkenyl, Cycloalkenyl, Alkinyl, Heterocyclyl, Alkylcarbonyl, Cycloalkylcarbonyl, Alkenylcarbonyl, Alkinylcarbonyl, Heterocyclylcarbonyl, Alkoxycarbonyl, Aryl, Hetaryl, Arylcarbonyl, Hetarylcarbonyl, Arylsulfonyl, Hetarylsulfonyl oder eine Gruppe C(R')-NOR";
R' Wasserstoff, Hydroxy, Cyano, Nitro, Amino, Halogen,
ggf. subst. Alkyl, Alkoxy, Alkylthio, Alkylamino, Dialkylamino, Alkenyl, Alkenyloxy, Alkenylthio, Alkenylamino, Alkinyl, Alkinyloxy, Alkinylthio, Alkinylamino, Cycloalkyl, Cycloalkoxy, Cycloalylthio, Cycloalkylamino, Cycloalkenyl, Cycloalkenyloxy, Cycloalkenylthio, Cycloalkenylamino, Heterocyclyl, Heterocyclyloxy, Heterocyclylthio, Heterocyclylamino, Aryl, Aryloxy, Arylthio, Arylamino, Heteroaryl, Heteroaryloxy, Heteroarylthio oder Heteroarylamino;
R" Wasserstoff,
ggf. subst. Alkyl, Cycloalkyl, Alkenyl, Alkinyl, Heterocyclyl, Aryl oder Heteroaryl,
R^{c} die unter R^{b} genannten Gruppen oder Hydroxy, Cyano, Nitro, Amino, Halogen,
ggf. subst. Alkoxy, Alkylthio, Alkylamino, Dialkylamino, Aryloxy, Arylthio, Arylamino, Hetaryloxy, Hetarylthio oder Hetarylamino;
oder
R^{b} und R^{c} gemeinsam mit dem C-Atom, an das sie gebunden sind ein carbocyclischer oder heterocyclischer Ring, durch Pinner-Umsetzung eines Acylcyanids der Formel IIA 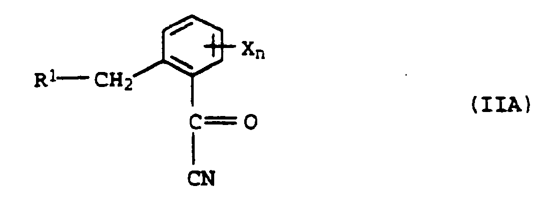
mit einem Alkohol und anschließende Umsetzung des bei der Pinner-Reaktion gebildeten Esters der Formel IVA, 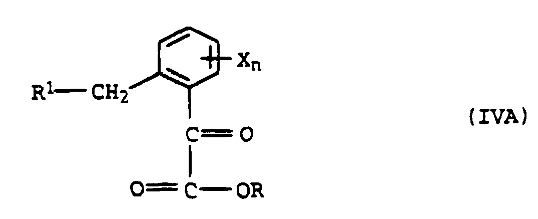
a) mit Hydroxylamin zum Oxim der Formel VA, 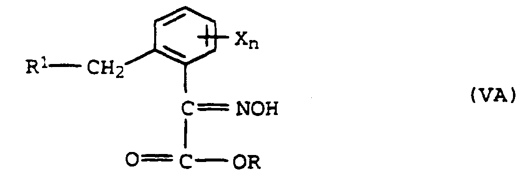 Methylierung von VA zum Oximether der Formel VIA 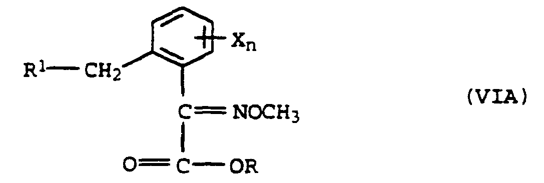 oder
b) mit O-Methylhydroxylamin zum Oximether der Formel VIA
und anschließende Umsetzung von VIA mit Methylamin, **dadurch gekennzeichnet, daß** man bei der Pinner-Reaktion einen Alkohol der Formel III
R-OH (III)
verwendet, dessen Siedepunkt oberhalb von 75°C liegt.

7. Verfahren zur Herstellung von α-Methoxyiminocarbonsäuremethylamiden der Formel IA gemäß Anspruch 6 - durch Pinner-Umsetzung eines Acylcyanids der Formel IIA 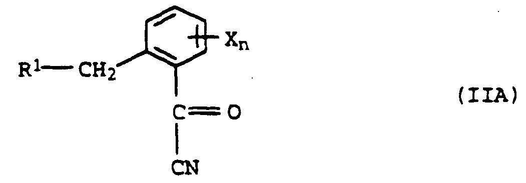 mit einem Alkohol und anschließende Umsetzung des bei der Pinner-Reaktion gebildeten Gemisches aus dem Ester der Formel IVA 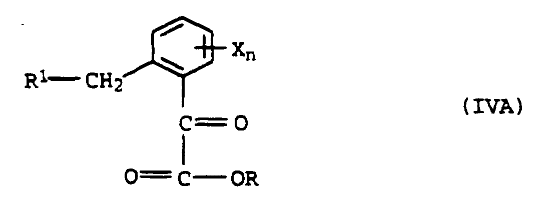 und dem Amid der Formel IV'A 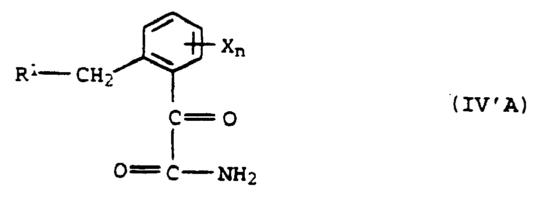
a) mit Hydroxylamin zum Oxim der Formel VA, 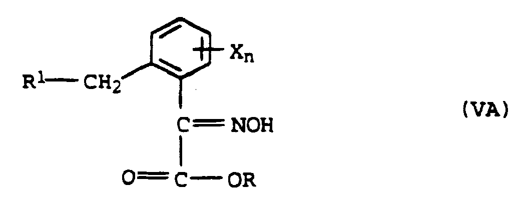 Methylierung von VA zum Oximether der Formel VIA 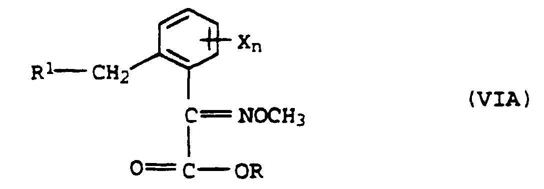 oder
b) mit O-Methylhydroxylamin zum Oximether der Formel VI
und anschließende Umsetzung von VIA mit Methylamin, **dadurch gekennzeichnet** - daß der man bei der Pinner-Reaktion einen Alkohol der Formel III
R-OH (III)
verwendet, dessen Siedepunkt oberhalb von 75°C liegt.

8. Verfahren zur Herstellung von α-Methoxyiminocarbonsäuremethylamiden der Formel IA nach Anspruch 6 oder 7, **dadurch gekennzeichnet, daß** man die Umsetzung zum Okim der Formel VA in Gegenwart desjenigen Alkohols III durchfuhrt, der bei der Pinner-Reaktion verwendet wurde.

9. Verfahren zur Herstellung von α-Methoxyiminocarbonsäuremethylamiden der Formel IA nach Anspruch 6 oder 7, **dadurch gekennzeichnet, daß** man die Umsetzung zum Oximether der Formel VIA in Gegenwart desjenigen Alkohols III durchfuhrt, der bei der Pinner-Reaktion verwendet wurde.

10. Verfahren nach den Ansprüchen 6 bis 9, **dadurch gekennzeichnet, daß** man einen Alkohol III verwendet, dessen Siedepunkt oberhalb von 90°C liegt.

11. Verbindungen der Formeln IVA und VIA:
(2-Chlormethyl)phenylglyoxylsäureethylester,
(2-Chlormethyl)phenylglyoxylsäure-n-propylester,
(2-Chlormethyl)phenylglyoxylsäure-iso-propylester,
(2-Chlormethyl)phenylglyoxylsäure-n-butylester,
(2-Chlormethyl)phenylglyoxylsäure-iso-butylester,
(2-Chlormethyl)phenylglyoxylsäurebut-2-ylester,
(2-Chlörmethyl)phenylglyoxylsäure-n-pentylester,
(2-Chlormethyl)phenylglyoxylsäure(3-methylbutyl)ester,
(2-Chlormethyl)phenylglyoxylsäure(2,2-dimethylpropyl)ester,
(2-Chlormethyl)phenylglyoxylsäure(1,1-dimethylpropyl)ester,
(2-Chlormethyl)phenylglyoxylsäurepent-2-ylester,
(2-Chlormethyl)phenylglyoxylsäurepent-3-ylester,
(2-Chlormethyl)phenylglyoxylsäure(2-methylbut-1-yl)-ester,
(2-Chlormethyl)phenylglyoxylsäure(3-methylbut-2-yl)-ester,
2-Methoxyimino-2-(2'-chlormethylphenyl)essigsäureethylester,
2-Methoxyimino-2-(2'-chlormethylphenyl)essigsäure-n-propylester,
2-Methoxyimino-2-(2'-chlormethylphenyl)essigsäure-iso-propylester,
2 -Methoxyimino-2-(2'-chlormethylphenyl)essigsäure-n-butylester,
2-Methoxyimino-2-(2'-chlormethylphenyl)essigsäure-iso-butylester,
2-Methoxyimino-2-(2'-chlormethylphenyl)essigsänrebut-2-ylester,
2-Methoxyimino-2-(2'-chlormethylphenyl)essigsäure-n-pentylester,
2-Methoxyimino-2-(2'-chlormethylphenyl)essigsäure(3-methylbutyl)ester,
2-Methoxyirnino-2-(2'-chlormethylphenyl)essigsäure(2,2-dimethylpropyl)ester,
2-Methoxyimino-2-(2'-chlormethylphenyl)essigsäure(1,1-dimethylpropyl)ester,
2-Methoxyimino-2-(2'-chlormethylphenyl)essigsäurepent-2-ylester,
2-Methoxyimino-2-(2'-chlormethylphenyl)essigsäurepent-3-ylester,
2-Methoxyimino-2-(2'-chlormethylphenyl)essigsäure(2-methylbut-1-yl)ester, und
2-Methoxyimino-2-(2'-chlormethylphenyl)essigsäure(3-methylbut-2-yl)ester.

## Claims

1. Process for preparing α-methoxyiminocarboxylic acid methylamides of the formula I 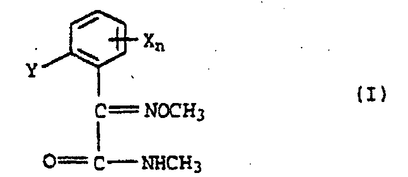 where
X is nitro, trifluoromethyl, halogen, C₁-C₄-alkyl or C₁-C₄-alkoxy,
n is 0 or an integer of from 1 to 4, where the X radicals can be different if n > 1, and where
Y is a C-organic radical,
by Pinner reaction of an acyl cyanide of the formula II 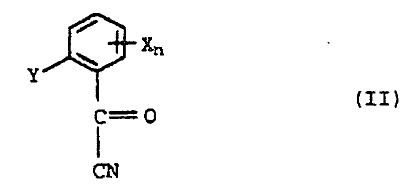 with an alcohol and subsequent reaction of the ester formed in the Pinner reaction, of the formula IV 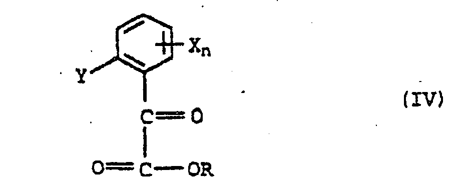
a) with hydroxylamine to give the oxime of the formula V 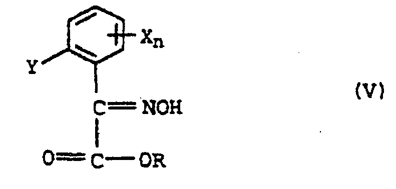 methylation of V to give the oxime ether of the formula VI 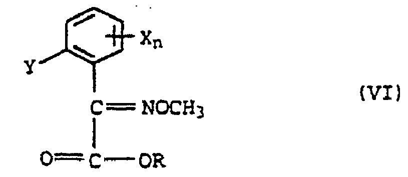 or
b) with O-methylhydroxylamine to give the oxime ether of the formula VI
and subsequent reaction of VI with methylamine, **characterized in that** it comprises using in the Pinner reaction an alcohol of the formula III
R-OH (III)
whose boiling point is above 75°C.

2. Process for preparing α-methoxyiminocarboxylic acid methylamides of the formula I according to claim 1, by Pinner reaction of an acyl cyanide of the formula II 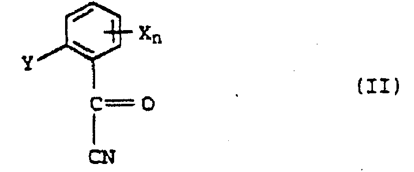 with an alcohol and subsequent reaction of the mixture formed in the Pinner reaction, of the ester of the formula IV 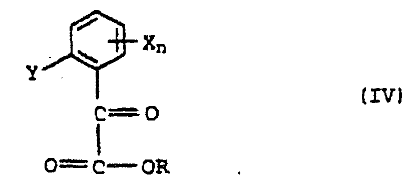 and the amide of the formula IV' 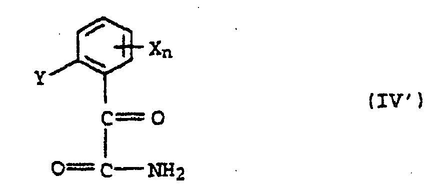
a) with hydroxylamine to give the oxime of the formula V 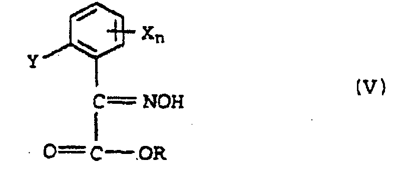 methylation of V to give the oxime ether of the formula VI 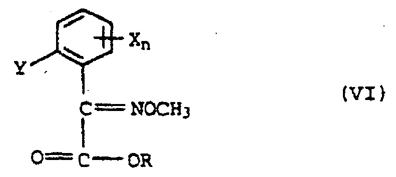 or
b) with O-methylhydroxylamine to give the oxime ether of the formula VI
and subsequent reaction of VI with methylamine, **characterized in that** it comprises using in the Pinner reaction an alcohol of the formula III
R-OH (III)
whose boiling point is above 75 °C.

3. Process for preparing α-methoxyiminocarboxylic acid methylamides of the formula I according to claim 1 or 2, **characterized in that** the reaction to give the oxime of the formula V is carried out in the presence of the alcohol III which was used in the Pinner reaction.

4. Process for preparing α-methoxyiminocarboxylic acid methylamides of the formula I according to claim 1 or 2, **characterized in that** the reaction to give the oxime ether of the formula VI is carried out in the presence of the alcohol III which was used in the Pinner reaction.

5. Process according to claims 1 to 4, **characterized in that** an alcohol III is used whose boiling point is above 90°C.

6. Process for preparing α-methoxyiminocarboxylic acid methylamides of the formula IA 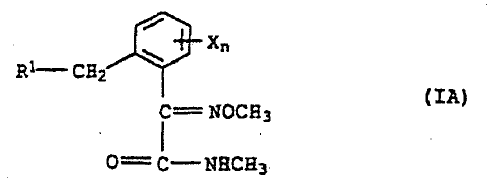 where the substituents and the index have the following meanings:
X is nitro, trifluoromethyl, halogen, C₁-C₄-alkyl or C₁-C₄-alkoxy,
n is 0 or an integer of from 1 to 4, where the X radicals can be different if n > 1,
R¹ is hydrogen, hydroxyl, mercapto, cyano, nitro, halogen, optionally substituted alkylsulfonyl, optionally substituted alkylsulfonyloxy, optionally substituted cycloalkyl, optionally substituted aryloxy, optionally substituted arylsulfonyl, optionally substituted arylsulfonyloxy, optionally substituted heterocyclyl or optionally substituted hetaryloxy, 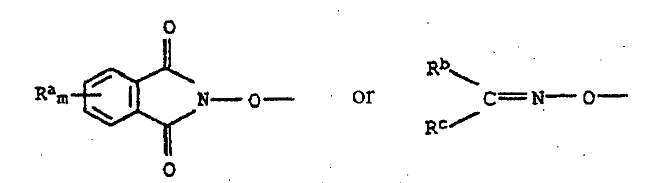
R^{a} is cyano, nitro, halogen, C₁-C₄-alkyl, C₁-C₄-haloalkyl, C₁-C₄-alkoxy or C₁-C₄-haloalkoxy,
m is 0 or an integer of from 1 to 4, where the R^{a} radicals can be different if m > 1,
R^{b} is hydrogen,
optionally substituted alkyl, cycloalkyl, alkenyl, cycloalkenyl, alkynyl, heterocyclyl, alkylcarbonyl, cycloalkylcarbonyl, alkenylcarbonyl, alkynylcarbonyl, heterocyclylcarbonyl, alkoxycarbonyl, aryl, hetaryl, arylcarbonyl, hetarylcarbonyl, arylsulfonyl, hetarylsulfonyl or a C(R')-NOR" group;
R' is hydrogen, hydroxyl, cyano, nitro, amino, halogen,
optionally substituted alkyl, alkoxy, alkylthio, alkylamino, dialkylamino, alkenyl, alkenyloxy, alkenylthio, alkenylamino, alkynyl, alkynyloxy, alkynylthio, alkynylamino, cycloalkyl, cycloalkoxy, cycloalkylthio, cycloalkylamino, cycloalkenyl, cycloalkenyloxy, cycloalkenylthio, cycloalkenylamino, heterocyclyl, heterocyclyloxy, heterocyclylthio, heterocyclylamino, aryl, aryloxy, arylthio, arylamino, heteroaryl, heteroaryloxy, heteroarylthio or heteroarylamino;
R" is hydrogen,
optionally substituted alkyl, cycloalkyl, alkenyl, alkynyl, heterocyclyl, aryl or heteroaryl,
R^{c} is a group mentioned under R^{b} or hydroxyl, cyano, nitro, amino, halogen,
optionally substituted alkoxy, alkylthio, alkylamino, dialkylamino, aryloxy, arylthio, arylamino, hetaryloxy, hetarylthio or hetarylamino;
or
R^{b} and R^{c}, together with the C atom to which they are bonded, are a carbocyclic or heterocyclic ring, by Pinner reaction of an acyl cyanide of the formula IIA 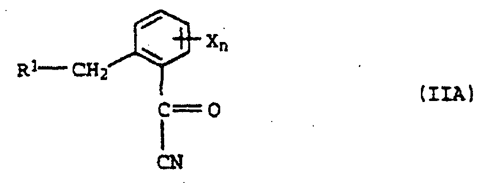 with an alcohol and subsequent reaction of the ester formed in the Pinner reaction, of the formula IVA 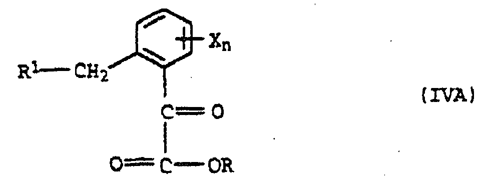
a) with hydroxylamine to give the oxime of the formula VA 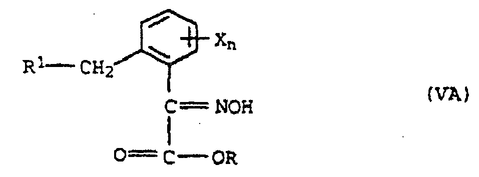 methylation of VA to give the oxime ether of the formula VIA 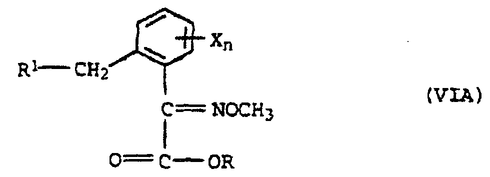 or
b) with O-methylhydroxylamine to give the oxime ether of the formula VIA
and subsequent reaction of VIA with methylamine, **characterized in that** it comprises using in the Pinner reaction an alcohol of the formula III
R-OH (III)
whose boiling point is above 75°C.

7. Process for preparing α-methoxyiminocarboxylic acid methylamides of the formula IA according to claim 6 - by Pinner reaction of an acyl cyanide of the formula IIA 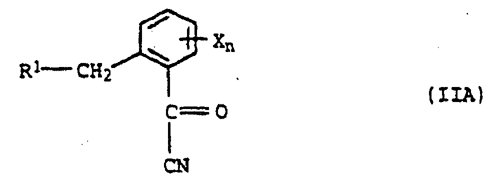 with an alcohol and subsequent reaction of the mixture formed in the Pinner reaction, of the ester of the formula IVA 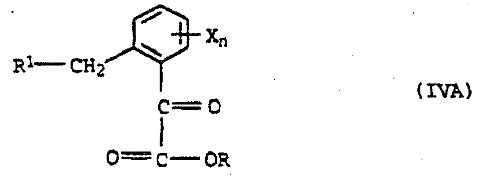 and the amide of the formula IV'A 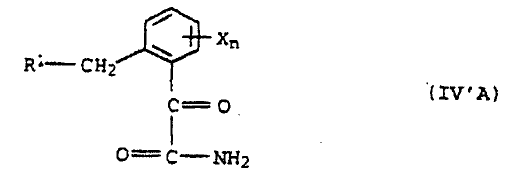
a) with hydroxylamine to give the oxime of the formula VA 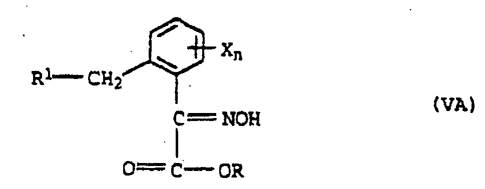 methylation of VA to give the oxime ether of the formula VIA 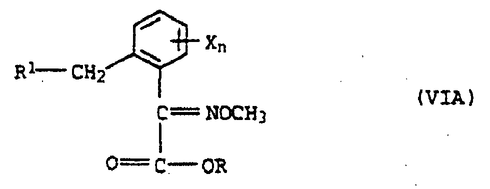 or
b) with O-methylhydroxylamine to give the oxime ether of the formula VI
and subsequent reaction of VIA with methylamine, **characterized in that** it comprises using in the Pinner reaction an alcohol of the formula III
R-OH (III)
whose boiling point is above 75°C.

8. Process for preparing α-methoxyiminocarboxylic acid methylamides of the formula IA according to claim 6 or 7, **characterized in that** the reaction to give the oxime of the formula VA is carried out in the presence of the alcohol III which was used in the Pinner reaction.

9. Process for preparing α-methoxyiminocarboxylic acid methylamides of the formula IA according to claim 6 or 7, **characterized in that** the reaction to give the oxime ether of the formula VIA is carried out in the presence of the alcohol III which was used in the Pinner reaction.

10. Process according to claims 6 to 9, **characterized in that** an alcohol III is used whose boiling point is above 90°C.

11. Compounds of the formulae IVA and VIA:
ethyl (2-chloromethyl)phenylglyoxylate,
n-propyl (2-chloromethyl)phenylglyoxylate,
isopropyl (2-chloromethyl)phenylglyoxylate,
n-butyl (2-chloromethyl)phenylglyoxylate,
isobutyl (2-chloromethyl)phenylglyoxylate,
but-2-yl (2-chloromethyl)phenylglyoxylate,
n-pentyl (2-chloromethyl)phenylglyoxylate,
3-methylbutyl (2-chloromethyl)phenylglyoxylate,
2,2-dimethylpropyl (2-chloromethyl)phenylglyoxylate,
1,1-dimethylpropyl (2-chloromethyl)phenylglyoxylate,
pent-2-yl (2-chloromethyl)phenylglyoxylate,
pent-3-yl (2-chloromethyl)phenylglyoxylate,
2-methylbut-1-yl (2-chloromethyl)phenylglyoxylate,
3-methylbut-2-yl (2-chloromethyl)phenylglyoxylate,
ethyl 2-methoxyimino-2-(2'-chloromethylphenyl)acetate,
n-propyl 2-methoxyimino-2-(2'-chloromethylphenyl)acetate,
isopropyl 2-methoxyimino-2-(2'-chloromethylphenyl)acetate,
n-butyl 2-methoxyimino-2-(2' -chloromethylphenyl)acetate,
isobutyl 2-methoxyimino-2-(2'-chloromethylphenyl)acetate,
but-2-yl 2-methoxyimino-2-(2'-chloromethylphenyl)acetate,
n-pentyl 2-methoxyimino-2-(2'-chloromethylphenyl)acetate,
3-methylbutyl 2-methoxyimino-2-(2'-chloromethylphenyl)acetate,
2,2-dimethylpropyl 2-methoxyimino-2-(2'-chloromethylphenyl)acetate,
1,1-dimethylpropyl 2-methoxyimino-2-(2'-chloromethylphenyl)acetate,
pent-2-yl 2-methoxyimino-2-(2'-chloromethylphenyl)acetate,
pent-3-yl 2-methoxyimino-2-(2'-chloromethylphenyl)acetate,
2-methylbut-1-yl 2-methoxyimino-2-(2'-chloromethylphenyl)acetate, and
3-methylbut-2-yl 2-methoxyimino-2-(2'-chloromethylphenyl)acetate.

## Revendications

1. Procédé pour la préparation de méthylamides d'acide α-méthoxyiminocarboxylique de formule 1, 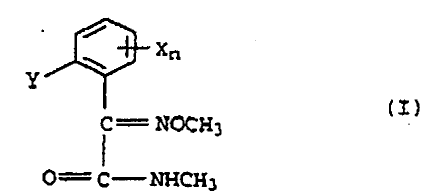 où
X désigne un groupe nitro, trifluorométhyle, halogéno, alkyle en C₁-C₄ ou alcoxy en C₁-C₄,
n vaut 0 ou un nombre entier de 1 à 4, tandis que les restes X peuvent être différents, lorsque n > 1, et où
Y signifie un reste C-organique,
par transformation de Pinner d'un acylcyanure de formule II 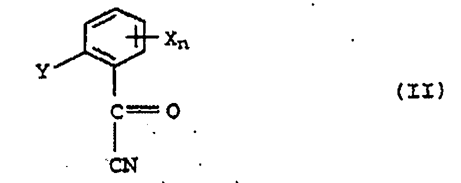 avec un alcool et ensuite réaction de l'ester de formule IV formé dans la réaction de Pinner 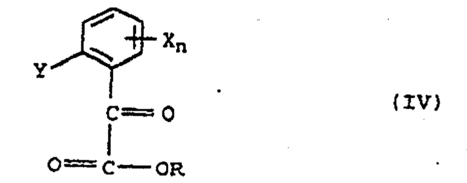
a) avec de l'hydroxylamine pour former l'oxime de formule V, 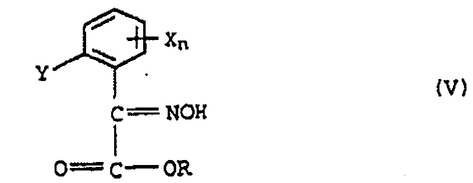 et méthylation de V pour donner l'éther d'oxime de formule VI 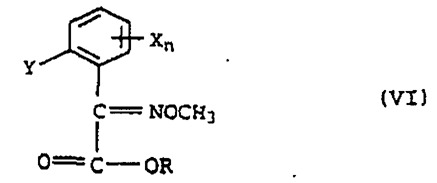 ou
b) avec de l'O-méthylhydroxylamine pour former l'éther d'oxime de formule VI et ensuite réaction de VI avec de la méthylamine, **caractérisé en ce qu'**on utilise dans la réaction de Pinner un alcool de formule III
R-OH (III)
dont le point d'ébullition est supérieur à 75°C.

2. Procédé pour la préparation de méthylamides d'acide α-méthoxyiminocarboxylique de formule 1 selon la revendication 1, par transformation de Pinner d'un acylcyanure de formule II 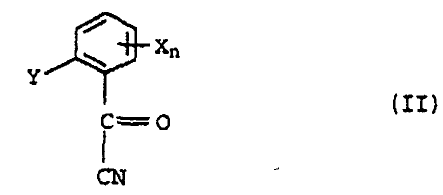 avec un alcool et ensuite réaction du mélange formé dans la réaction de Pinner à partir de l'ester de formule IV 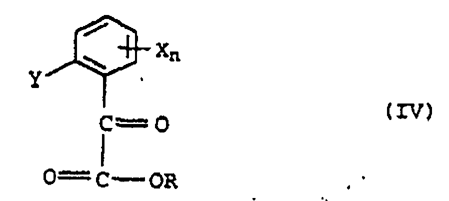 et de l'amide de formule IV' 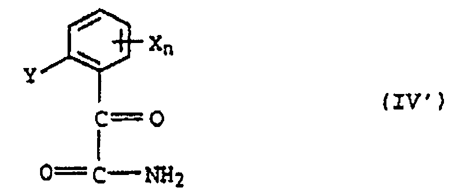
a) avec de l'hydroxylamine pour former l'oxime de formule V, 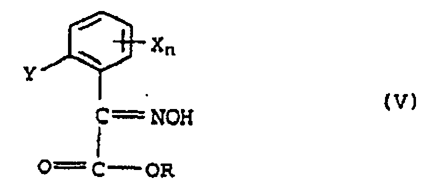 et méthylation de V pour donner l'éther d'oxime de formule VI 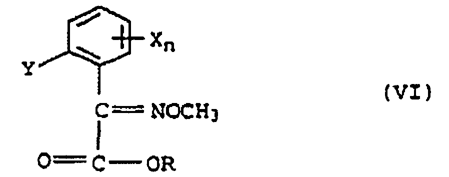 ou
b) avec de l'O-méthylhydroxylamine pour former l'éther d'oxime de formule VI et ensuite réaction de VI avec de la méthylamine, **caractérisé en ce qu'**on utilise dans la réaction de Pinner un alcool de formule III
R-OH (III)
dont le point d'ébullition est supérieur à 75°C.

3. Procédé pour la préparation de méthylamides d'acide α-méthoxyiminocarboxylique de formule I selon la revendication 1 ou 2,
**caractérisé en ce qu'**on effectue la conversion en l'oxime de formule V en présence de l'alcool III qui a été utilisé dans la réaction de Pinner.

4. Procédé pour la préparation de méthylamides d'acide α-méthoxyiminocarboxylique de formule I selon la revendication 1 ou 2, **caractérisé en ce qu'**on effectue la conversion en éther d'oxime de formule VI en présence de l'alcool III qui a été utilisé dans la réaction de Pinner.

5. Procédé selon les revendications 1 à 4, **caractérisé en ce qu'**on emploie un alcool III dont le point d'ébullition est supérieur à 90°C.

6. Procédé pour la préparation de méthylamides d'acide α-méthoxyiminocarboxylique de formule IA 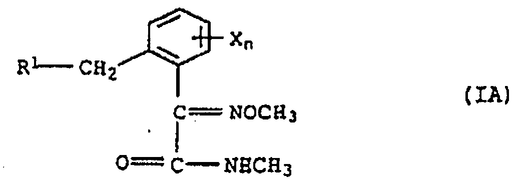 dans laquelle les substituants et l'indice ont la signification suivante :
X nitro, trifluorométhyle, halogéno, alkyle en C₁-C₄ ou alcoxy en C₁-C₄,
n 0 ou un nombre entier de 1 à 4, tandis que les restes X peuvent être différents lorsque n > 1,
R¹ hydrogène, hydroxy, mercapto, cyano, nitro, halogéno,
alkylsulfonyle éventuellement substitué, alkylsulfonyloxy éventuellement substitué, cycloalkyle éventuellement substitué, aryloxy éventuellement substitué, arylsulfonyle éventuellement substitué, arylsulfonyloxy éventuellement substitué, hétérocyclyle éventuellement substitué ou hétaryloxy éventuellement substitué, 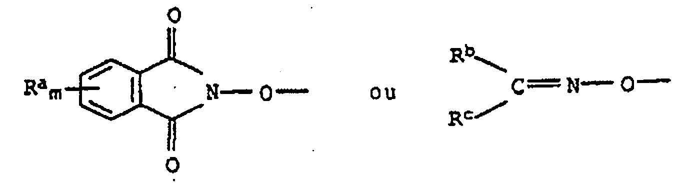
R^{a} cyano, nitro, halogéno, alkyle en C₁-C₄, halogénoalkyle en C₁-C₄, alcoxy en C₁-C₄ ou halogénoalcoxy en C₁-C₄,
m 0 ou un nombre entier de 1 à 4, tandis que les restes R^{a} peuvent être différents lorsque m > 1,
R^{b} hydrogène,
alkyle, cycloalkyle, alcényle, cycloalcényle, alcynyle, hétérocyclyle, alkylcarbonyle, cycloalkylcarbonyle, alcénylcarbonyle, alcynylcarbonyle, hétérocyclylcarbonyle, alcoxycarbonyle, aryle, hétaryle, arylcarbonyle, hétarylcarbonyle, arylsulfonyle, hétarylsulfonyle ou un groupe C(R')=NOR", éventuellement substitués,
R' hydrogène, hydroxy, cyano, nitro, amino, halogéno,
alkyle, alcoxy, alkylthio, alkylamino, dialkylamino, alcényle, alcényloxy, alcénylthio, alcénylamino, alcynyle, alcynyloxy, alcynylthio, alcynylamino, cycloalkyle, cycloalcoxy, cycloalkylthio, cycloalkylamino, cycloalcényle, cycloalcényloxy, cycloalcénylthio, cycloalcénylamino, hétérocyclyle, hétérocyclyloxy, hétérocyclylthio, hétérocyclylamino, aryle, aryloxy, arylthio, arylamino, hétéroaryle, hétéroaryloxy, hétéroarylthio ou hétéroarylamino, éventuellement substitués,
R" hydrogène,
alkyle, cycloalkyle, alcényle, alcynyle, hétérocyclyle, aryle ou hétéroaryle, éventuellement substitués,
R^{c} les groupes mentionnés sous R^{b} ou
hydroxy, cyano, nitro, amino, halogéno,
alcoxy, alkylthio, alkylamino, dialkylamino, aryloxy, arylthio, arylamino, hétaryloxy, hétarylthio ou hétarylamino, éventuellement substitués,
ou R^{b} et R^{c}, conjointement avec l'atome de carbone auquel ils sont liés, forment un noyau carbocyclique ou hétérocyclique, par réaction de Pinner d'un cyanure d'acyle de formule IIA 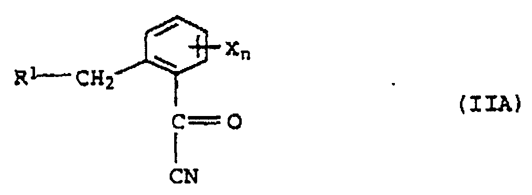 avec un alcool et réaction ultérieure de l'ester de formule IVA formé dans la réaction de Pinner, 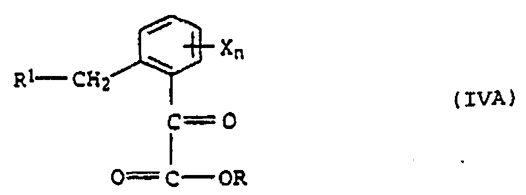
a) avec de l'hydroxylamine pour former l'oxime de formule VA, 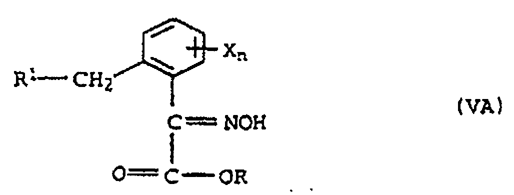 et méthylation de VA pour former l'éther d'oxime de formule VIA 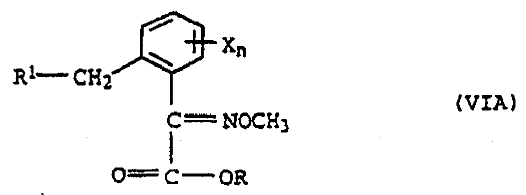 ou
b) avec de l'O-méthylhydroxylamine pour former l'éther d'oxime de formule VIA
et réaction ultérieure de VIA avec de la méthylamine, **caractérisé en ce qu'**on utilise dans la réaction de Pinner un alcool de formule III
R-OH (III)
dont le point d'ébullition est supérieur à 75°C.

7. Procédé pour la préparation de méthylamides d'acide α-méthoxyiminocarboxylique de formule IA selon la revendication 6, par conversion de Pinner d'un cyanure d'acyle de formule IIA 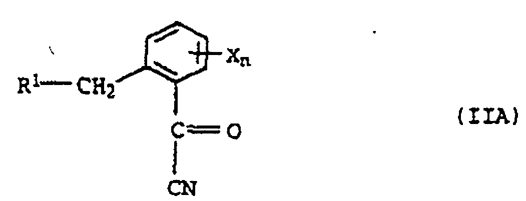 avec un alcool et réaction ultérieure du mélange, formé dans la réaction de Pinner, de l'ester de formule IVA 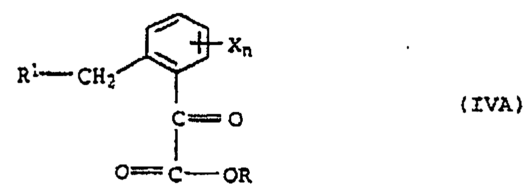 et de l'amide de formule IV'A 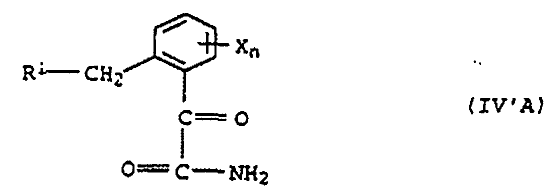
a) avec de l'hydroxylamine pour former l'oxime de formule VA 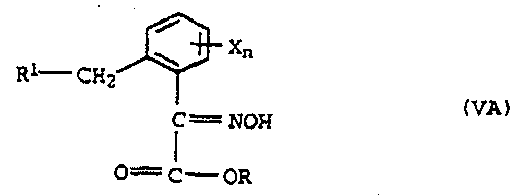 et méthylation de VA pour former l'éther d'oxime de formule VIA 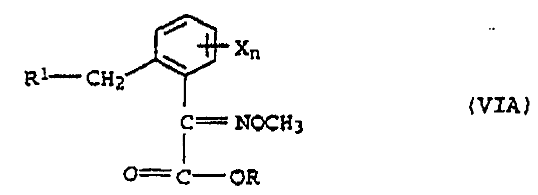 ou
b) avec de l'O-méthylhydroxylamine pour former l'éther d'oxime de formule VI et ensuite réaction de VIA avec de la méthylamine, **caractérisé en ce qu'**on utilise dans la réaction de Pinner un alcool de formule III
R-OH (III)
dont le point d'ébullition est supérieur à 75°C.

8. Procédé pour la préparation de méthylamides d'acide α-méthoxyiminocarboxylique de formule IA selon la revendication 6 ou 7, **caractérisé en ce qu'**on effectue la conversion en oxime de formule VA en présence de l'alcool III qui a été utilisé dans la réaction de Pinner.

9. Procédé pour la préparation de méthylamides d'acide α-méthoxyiminocarboxylique de formule IA selon la revendication 6 ou 7, **caractérisé en ce qu'**on effectue la conversion en éther d'oxime de formule VIA en présence de l'alcool III qui a été utilisé dans la réaction de Pinner.

10. Procédé selon les revendications 6 à 9, **caractérisé en ce qu'**on utilise un alcool III dont le point d'ébullition est supérieur à 90°C.

11. Composés des formules IVA et VIA :
ester éthylique de l'acide (2-chlorométhyl)phénylglyoxylique,
ester n-propylique de l'acide (2-chlorométhyl)phénylglyoxylique,
ester isopropylique de l'acide (2-chlorométhyl)phénylglyoxylique,
ester n-butylique de l'acide (2-chlorométhyl)phénylglyoxylique,
ester isobutylique de l'acide (2-chlorométhyl)phénylglyoxylique,
ester but-2-ylique de l'acide (2-chlorométhyl)phénylglyoxylique,
ester n-pentylique de l'acide (2-chlorométhyl)phénylglyoxylique,
ester 3-méthylbutylique de l'acide (2-chlorométhyl)phénylglyoxylique,
ester 2,2-diméthylpropylique de l'acide (2-chlorométhyl)phénylglyoxylique,
ester 1,1-diméthylpropylique de l'acide (2-chlorométhyl)phénylglyoxylique,
ester pent-2-ylique de l'acide (2-chlorométhyl)phénylglyoxylique,
ester pent-3-ylique de l'acide (2-chlorométhyl)phénylglyoxylique,
ester 2-méthylbut-1-ylique de l'acide (2-chlorométhyl)phénylglyoxylique,
ester 3-méthylbut-2-ylique de l'acide (2-chlorométhyl)phénylglyoxylique,
ester éthylique de l'acide 2-méthoxyimino-2-(2'-chlorométhylphényl)acétique,
ester n-propylique de l'acide 2-méthoxyimino-2-(2'-chlorométhylphényl)acétique,
ester iso-propylique de l'acide 2-méthoxyimino-2-(2'-chlorométhylphényl)acétique,
ester n-butylique de l'acide 2-méthoxyimino-2-(2'-chlorométhylphényl)acétique,
ester iso-butylique de l'acide 2-méthoxyimino-2-(2'-chlorométhylphényl)acétique,
ester but-2-ylique de l'acide 2-méthoxyimino-2-(2'-chlorométhylphényl)acétique,
ester n-pentylique de l'acide 2-méthoxyimino-2-(2'-chlorométhylphényl)acétique,
ester 3-méthylbutylique de l'acide 2-méthoxyimino-2-(2'-chlorométhylphényl)acétique,
ester 2,2-diméthylpropylique de l'acide 2-méthoxyimino-2-(2'-chlorométhylphényl)acétique,
ester 1,1-diméthylpropylique de l'acide 2-méthoxyimino-2-(2'-chlorométhylphényl)acétique,
ester pent-2-ylique de l'acide 2-méthoxyimino-2-(2'-chlorométhylphényl)acétique,
ester pent-3-ylique de l'acide 2-méthoxyimino-2-(2'-chlorométhylphényl)acétique,
ester 2-méthylbut-1-ylique de l'acide 2-méthoxyimino-2-(2'-chlorométhylphényl)acétique, et
ester 3-méthylbut-2-ylique de l'acide 2-méthoxyimino-2-(2'-chlorométhylphényl)acétique.
